# EUROPEAN PATENT SPECIFICATION

(11) **EP 4 062 974 B1**
(45) Date of publication and mention of the grant of the patent: **08.05.2024**
(21) Application number: 22160354.1
(22) Date of filing: 04.03.2022
(51) Int. Cl.: A61P 35/00, C07K 7/06, C07K 14/47

(54) **PHARMACEUTICAL COMPOSITION FOR PREVENTING OR TREATING CANCER**
PHARMAZEUTISCHE ZUSAMMENSETZUNG ZUR VORBEUGUNG ODER BEHANDLUNG VON KREBS
COMPOSITION PHARMACEUTIQUE POUR LA PRÉVENTION OU LE TRAITEMENT DU CANCER

(30) Priority: 22.03.2021 KR 20210036803; 18.02.2022 WO PCT/KR2022/002399; 25.02.2022 WO PCT/KR2022/002762
(43) Date of publication of application: 28.09.2022
(73) Proprietor: L-Base Co.,Ltd., Seoul 04778 (KR)
(72) Inventor: JEON, Do Yong, 04778 Seoul (KR)
(74) Representative: Dehns

(56) References cited:
- WO-A1-2021/194228
- WO-A2-2011/048390
- US-A1- 2018 057 532
- YOUNGMI KIM ET AL: "The pentapeptide Gly-Thr-Gly-Lys-Thr confers sensitivity to anti-cancer drugs by inhibition of CAGE binding to GSK3? and decreasing the expression of cyclinD1", ONCOTARGET, vol. 8, no. 8, 21 February 2017 (2017-02-21), pages 13632-13651, XP055756625, DOI: 10.18632/oncotarget.14621
- MINJEONG YEON ET AL: "CAGE Binds to Beclin1, Regulates Autophagic Flux and CAGE-Derived Peptide Confers Sensitivity to Anti-cancer Drugs in Non-small Cell Lung Cancer Cells", FRONTIERS IN ONCOLOGY, vol. 8, 10 December 2018 (2018-12-10), XP055756617, DOI: 10.3389/fonc.2018.00599
- KOEHLER MICHAEL F T ET AL: "Albumin affinity tags increase peptide half-life in vivo", BIOORGANIC & MEDICINAL CHEMISTRY LETTERS, ELSEVIER, AMSTERDAM NL, vol. 12, no. 20, 11 June 2017 (2017-06-11) , pages 2883-2886, XP085060910, ISSN: 0960-894X, DOI: 10.1016/S0960-894X(02)00610-8
- KINOSHITA YUHEI ET AL: "Study of the structure-activity relationship of polymyxin analogues", BIOORGANIC & MEDICINAL CHEMISTRY LETTERS, ELSEVIER, AMSTERDAM NL, vol. 28, no. 16, 22 March 2018 (2018-03-22), pages 2713-2716, XP085457705, ISSN: 0960-894X, DOI: 10.1016/J.BMCL.2018.03.028
- WERLE M ET AL: "Strategies to improve plasma half life time of peptide and protein drugs", AMINO ACIDS ; THE FORUM FOR AMINO ACID AND PROTEIN RESEARCH, SPRINGER-VERLAG, VI, vol. 30, no. 4, 20 April 2006 (2006-04-20) , pages 351-367, XP019430805, ISSN: 1438-2199, DOI: 10.1007/S00726-005-0289-3

## Description

### BACKGROUND

### 1. Field of the Invention

The present invention relates to an analog compound of the oligopeptide AQTGTGKT, a pharmaceutical composition for preventing or treating cancer including the same as an active ingredient, and a preparation method thereof.

### 2. Discussion of Related Art

Currently, even though the cancer treatment effect is improving due to the development of early diagnosis methods for cancer and the continuous development of new anticancer therapies, the cancer is still considered as critical disease due to its ranking in the first or second place among the causes of death in Korea. Most of the anticancer drugs currently used are based on chemotherapy, which is pointed out as a problem in cancer treatment because the pharmacological action varies according to the type of cancer, and side effects due to toxicity variously appear.

Since existing anticancer drugs penetrate not only cancer cells but also normal cells and damage the function and activity of normal cells, the existing anticancer drugs may also cause side effects such as bone marrow dysfunction, gastrointestinal disturbances, and alopecia, and show major problems in cancer treatment, such as multi-drug resistance to anticancer drugs by long-term chemotherapy. Therefore, studies on the development of innovative anticancer drugs capable of solving these serious problems of existing anticancer drugs are being actively conducted.

Meanwhile, even though antibodies targeting specific tumor antigens of tumor cells have been developed, antibodies have problems such as concerns about immune responses and low efficiency of penetration into tissues. In contrast, unlike antibodies, peptides have an advantage of less concerns about immune responses and easy penetration into tissues due to their small molecular weight, and peptide-based anticancer drugs targeting tumor antigens can selectively act on tumors, so that it is expected to have a less side effects such as damage to cells. However, in spite of these advantages, peptides have been used only for very limited carcinomas, and in particular, have a problem in that it is difficult to exert the effects of the peptides because the peptides are degraded in a short time immediately after being administered to humans.

Although it is possible to achieve an improvement in yield, an increase in in vivo activity, an increase in affinity of peptides for receptors, a delay in protein degradation, and the like by modifying peptides through amidation, esterification, acylation, acetylation, PEGylation, cyclization, alkylation or the like, it is not guaranteed that a desired effect will be improved even by the modification, and there is a risk in that the peptide's original in vivo activity may be lost or unexpected side effects may occur. Therefore, studies on the modification of peptides to minimize these negative effects and simultaneously maximize the desired effects have been actively conducted.

US2018/057532A1 reports a peptide having anticancer activity, and refers to a peptide which has the amino acid sequence AQTGTGKT.

Kim et al. (Oncotarget, 2017, Vol. 8, No.8, pages 13632-13651) report that the pentapeptide Gly-Thr-Gly-Lys-Thr confers sensitivity to anti-cancer drugs by inhibition of CAGE binding to GSKβ and decreasing the expression of cyclinD1.

Yeon et al. (Frontiers in Oncology, 2018, Vol. 8, Article 599) report that CAGE binds to Beclin1, regulates autophagic flux and CAGE-derived peptide confers sensitivity to anti-cancer drugs in non-small cell lung cancer cells.

WO2011/048390A2 reports compounds based around tetrapeptide, tripeptide and dipeptide moieties and corresponding peptoid moieties, and related methods and pharmaceutical compositions for use in treatment of cancer, inflammatory diseases, and other disorders.

Koehler et al. (Bioorganic & Medicinal Chemistry Letters, 2002, Vol. 12, No. 20, pages 2883-2886) report that albumin affinity tags increase peptide half-life in vivo.

Kinoshita et al. (Bioorganic & Medicinal Chemistry Letters, 2018, Vol. 28, No. 16, pages 2713-2716) report a study of the structure-activity relationship of polymyxin analogues).

Werle and Bernkop-Schnürch (Amino Acids, 2006, Vol. 30, No. 4, pages 351-367) review strategies to improve plasma half life time of peptide and protein drugs.

### [Related Art Document]

### [Patent Document]

(Patent Document 1) KR10-2073144 B1

### SUMMARY OF THE INVENTION

The present invention has been made in an effort to solve the problems in the related art as described above, and as a result of intensive studies to further improve the anticancer effect of an oligopeptide alanine-glutamine-threonine-glycine-threonine-glycine-lysine-threonine (AQTGTGKT) and its half-life in blood, the present invention was completed. In particular, it was confirmed that an amidated analog compound of the AQTGTGKT peptide has an excellent anticancer effect and an increased half-life.

Embodiments of the present invention are described in the claims.

An objective of the present invention is to provide a compound represented by the following General Formula:

[General Formula] X-AQTGTGKT

In General Formula, A is alanine, Q is glutamine, T is threonine, G is glycine, and K is lysine, and
X is one or more selected from the group consisting of

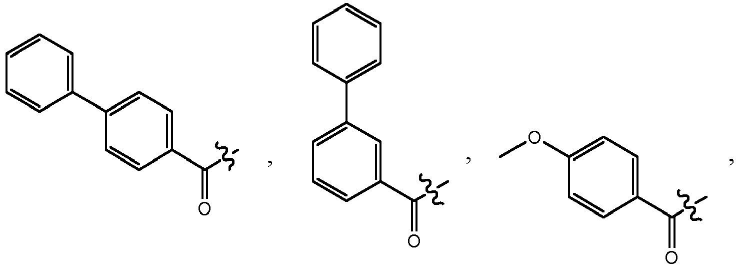

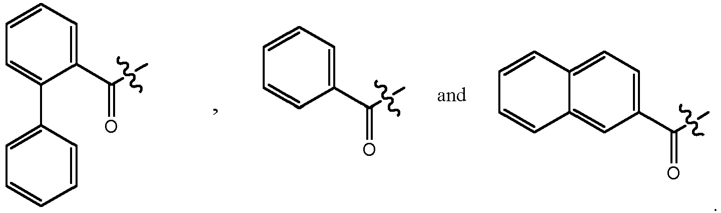

Another objective of the present invention is to provide a pharmaceutical composition for preventing or treating cancer, including the compound represented by General Formula as an active ingredient.

Still another objective of the present invention is to provide a method for preparing the compound represented by General Formula.

In order to achieve the objectives of the present invention as described above, the present invention provides a compound represented by the following General Formula:

[General Formula] X-AQTGTGKT

In General Formula, A is alanine, Q is glutamine, T is threonine, G is glycine, and K is lysine, and
the X is one or more selected from the group consisting of

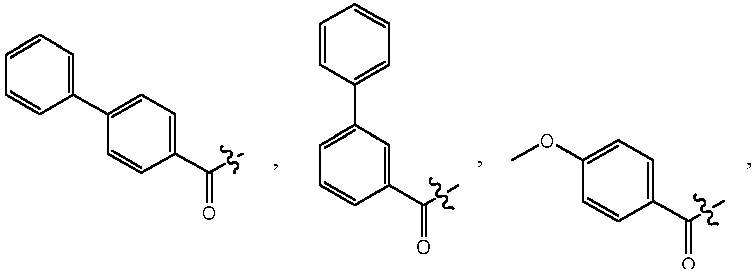

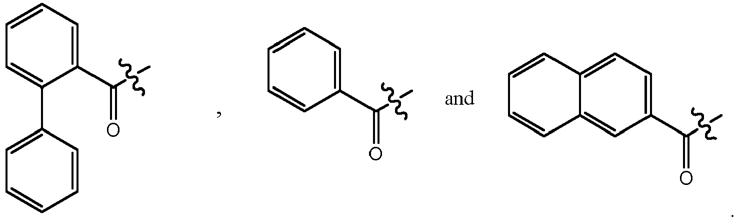

Further, the present invention provides a pharmaceutical composition for preventing or treating cancer, including a compound represented by the following General Formula or a pharmaceutically acceptable salt thereof as an active ingredient:

[General Formula] X-AQTGTGKT

In General Formula, A is alanine, Q is glutamine, T is threonine, G is glycine, and K is lysine, and
the X is one or more selected from the group consisting of

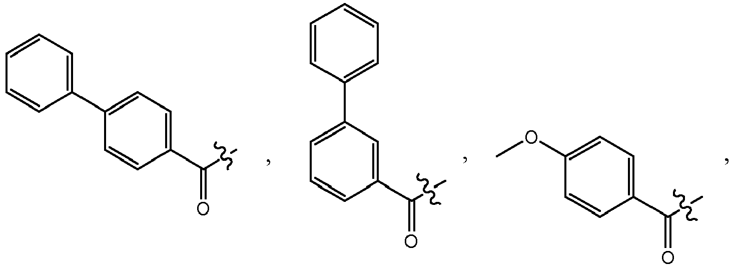

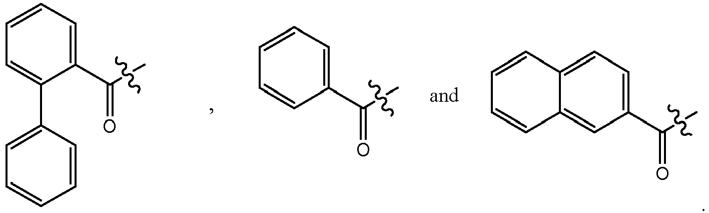

Further, the present invention provides a compound for use in the prevention or treatment of cancer, wherein the compound is represented by the following General Formula:

[General Formula] X-AQTGTGKT

(in General Formula,
A is alanine, Q is glutamine, T is threonine, G is glycine, and K is lysine, and
the X is one or more selected from the group consisting of

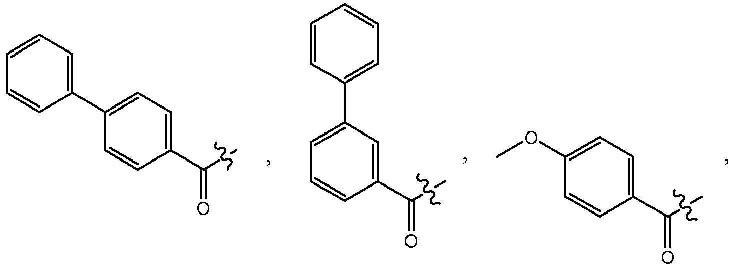

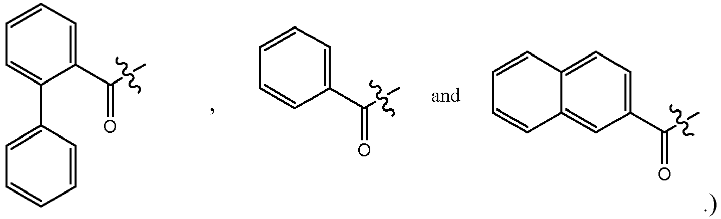

In an exemplary embodiment of the present invention, the cancer may be a cancer selected from the group consisting of lung cancer, breast cancer, blood cancer, colorectal cancer, pancreatic cancer, and combinations thereof, but is not limited thereto.

In another exemplary embodiment of the present invention, the lung cancer may be non-small cell lung cancer, but is not limited thereto.

In yet another exemplary embodiment, the blood cancer may be selected from the group consisting of leukemia, lymphoma, multiple myeloma, and combinations thereof, but is not limited thereto.

In an exemplary embodiment of the present invention, the cancer may be lung cancer, but is not limited thereto.

In another exemplary embodiment of the present invention, the cancer may be breast cancer, but is not limited thereto.

In still another exemplary embodiment of the present invention, the cancer may be blood cancer, but is not limited thereto.

In yet another exemplary embodiment of the present invention, the X is one or more selected from the group consisting of 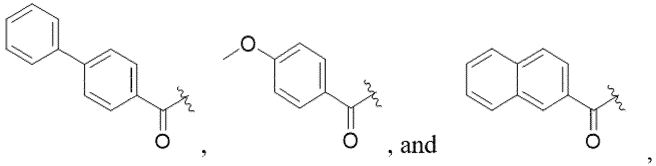 and the cancer may be pancreatic cancer, but is not limited thereto.

In yet another exemplary embodiment of the present invention, the cancer may be colorectal cancer, but is not limited thereto.

In an exemplary embodiment of the present invention, when the X is 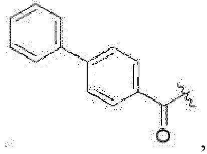 the half-life of the compound in human blood may be 100 minutes to 150 minutes, but is not limited thereto.

In another exemplary embodiment of the present invention, when the X is one or more selected from the group consisting of 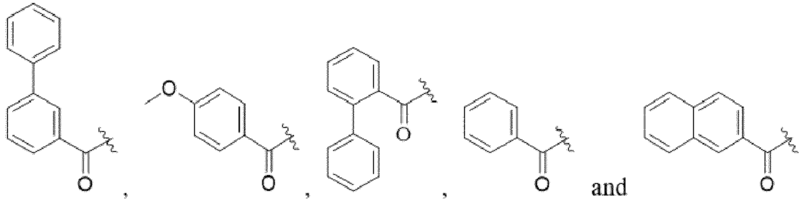 , the half-life of the compound in human blood may be 45 minutes to 70 minutes, but is not limited thereto.

Further, the present invention provides a method for preparing an oligopeptide X-AQTGTGKT, the method including: the following steps:
(A is alanine, Q is glutamine, T is threonine, G is glycine, and K is lysine, and
the X is one selected from the group consisting of 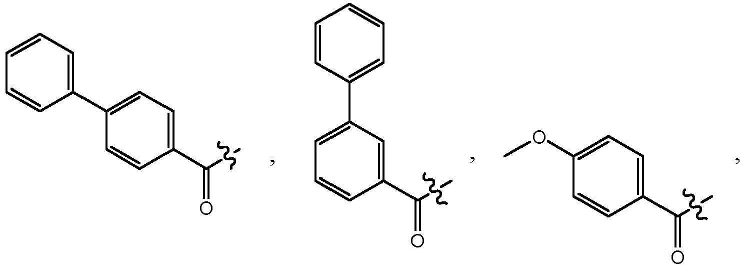 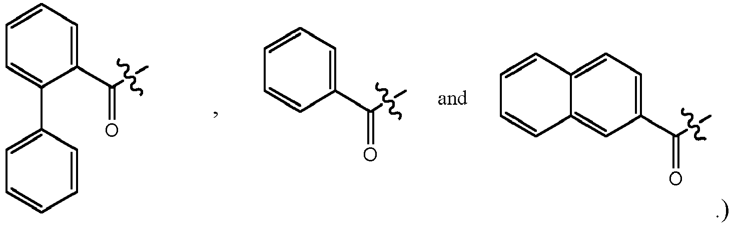
   (1) synthesizing each of TG and KT;
   (2) synthesizing TGKT by combining TG and KT;
   (3) synthesizing TGTGKT by bonding TG to the N-terminal of the TGKT;
   (4) synthesizing QTGTGKT by bonding Q to the N-terminal of the TGTGKT; and
   (5) synthesizing X-AQTGTGKT by bonding an alanine derivative (X-A) to the N-terminal of the QTGTGKT.

### BRIEF DESCRIPTION OF THE DRAWINGS

The above and other objectives, features and advantages of the present invention will become more apparent to those of ordinary skill in the art by describing in detail exemplary embodiments thereof with reference to the accompanying drawings, in which:

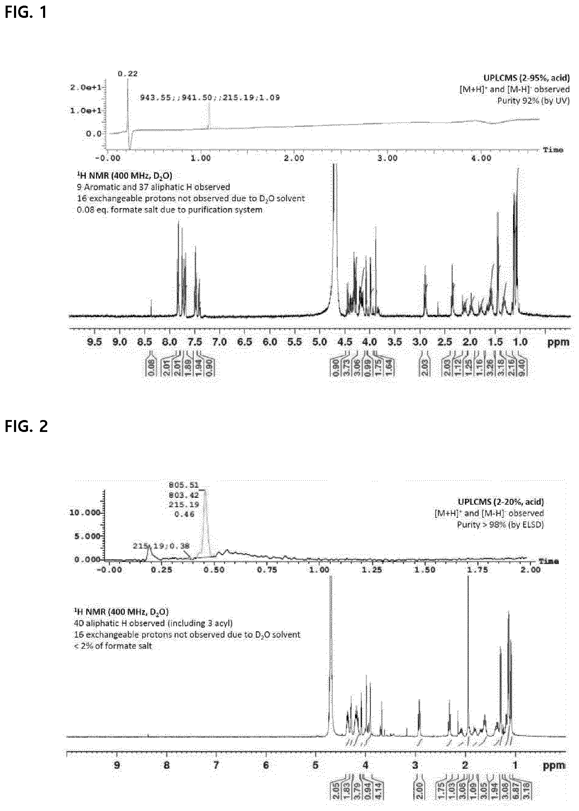

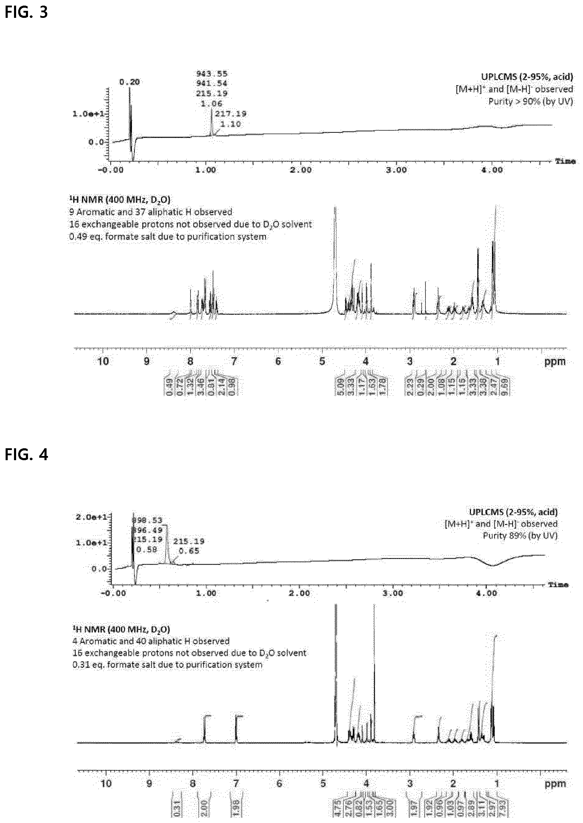

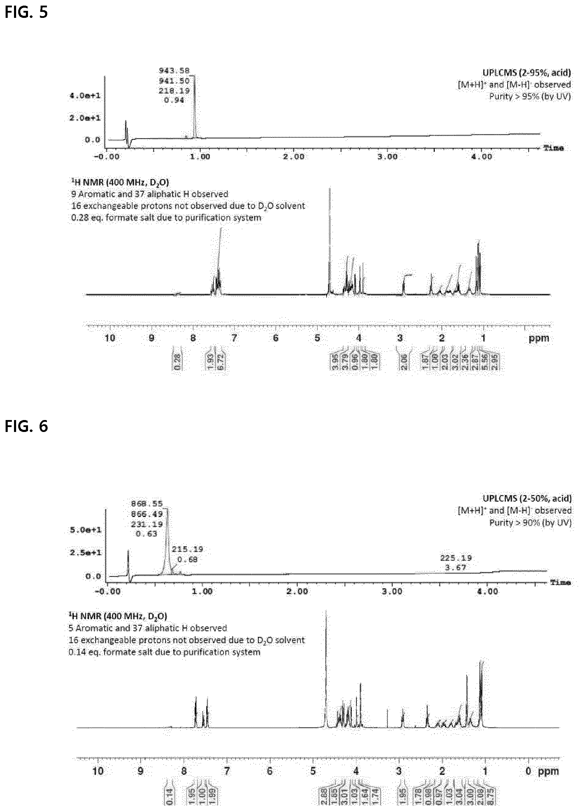

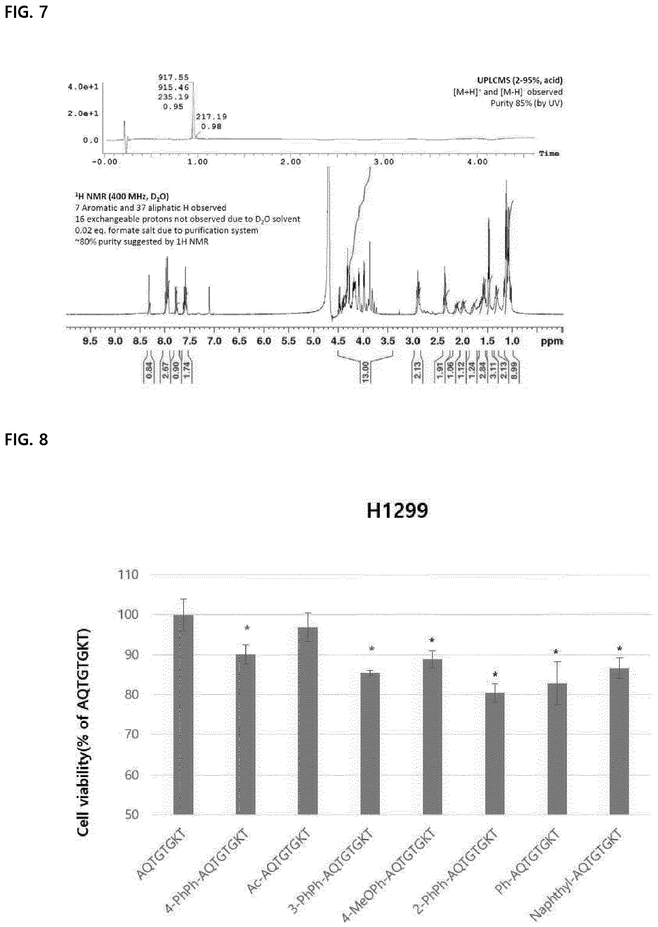

FIGS. 1 to 7 are views illustrating the UPLC-MS (top) and ¹H NMR (bottom) results for identifying the compound according to the present invention and confirming the structure of the compound, and FIG. 1 illustrates the results of measuring 4-PhPh-AQTGTGKT, FIG. 2 illustrates the results of measuring Ac-AQTGTGKT (Ac-AQTGTGKT is not a compound of the present invention), FIG. 3 illustrates the results of measuring 3-PhPh-AQTGTGKT, FIG. 4 illustrates the results of measuring 4-MeOPh-AQTGTGKT, FIG. 5 illustrates the results of measuring 2-PhPh-AQTGTGKT, FIG. 6 illustrates the results of measuring Ph-AQTGTGKT, and FIG. 7 illustrates the results of measuring Naphthyl-AQTGTGKT;
FIG. 8 is a view illustrating the results of analyzing the effect of suppressing cell viability according to treatment with the compound of the present invention in a lung cancer cell line H1299 by CTG assay (*p<0.05, **p<0.01, ***p<0.001; hereinafter, the same below);
FIG. 9 is a view illustrating the results of analyzing the effect of suppressing cell viability according to treatment with the compound of the present invention in a lung cancer cell line H1975 by CTG assay;
FIG. 10 is a view illustrating the results of analyzing the effect of suppressing cell viability according to treatment with the compound of the present invention in a lung cancer (papillary adenocarcinoma) cell line H820 by MTT assay;
FIG. 11a is a view illustrating the results of analyzing the effect of suppressing cell viability according to treatment with the compound of the present invention in a breast cancer cell line MDA-MB-231 by CTG assay;
FIG. 11b is a view illustrating the results of analyzing the effect of suppressing cell viability according to treatment with the compound of the present invention in a breast cancer cell line HCC1937 by MTT assay;
FIG. 12 is a view illustrating the results of analyzing the effect of suppressing cell viability according to treatment with the compound of the present invention in a blood cancer cell line Jurkat clone E6-1 by MTT assay;
FIG. 13 is a view illustrating the results of analyzing the effect of suppressing cell viability according to treatment with the compound of the present invention in a pancreatic cancer cell line CFPAC-1 by MTT assay;
FIG. 14 is a view illustrating the results of analyzing the effect of suppressing cell viability according to treatment with the compound of the present invention in a colorectal cancer cell line HT29 by CTG assay;
FIG. 15 is a view illustrating the results of analyzing tumor growth suppression according to treatment with the compound of the present invention after tumors are formed by inoculating nude mice with a lung cancer cell line H820;
FIG. 16 is a view illustrating the results of analyzing tumor growth suppression according to treatment with the compound of the present invention after tumors are formed by inoculating nude mice with a lung cancer cell line H1975;
FIG. 17 is a view illustrating the results of analyzing tumor growth suppression according to treatment with the compound of the present invention after tumors are formed by inoculating nude mice with a breast cancer cell line HCC1806;
FIG. 18 is a view illustrating the results of analyzing tumor volume suppression according to treatment with the compound of the present invention after tumors are formed by inoculating nude mice with a colorectal cancer cell line CT26; and
FIGS. 19 to 26 are views illustrating the results of measuring the residual amount of each compound according to the present invention in human blood over time.

### DETAILED DESCRIPTION OF EXEMPLARY EMBODIMENTS

The present inventors newly synthesized amidation analogs of an oligopeptide AQTGTGKT were newly synthesized, and confirmed that these amidation analogs exhibited an excellent anticancer effect (see Example 2) and excellent stability in blood (see Example 3), thereby completing the present invention. Thus, the present invention may provide a compound represented by the following General Formula:

[General Formula] X-AQTGTGKT

In General Formula, A is alanine, Q is glutamine, T is threonine, G is glycine, K is lysine, and the X is one or more selected from the group consisting of

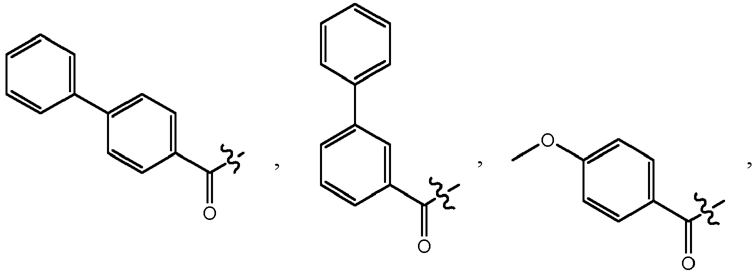

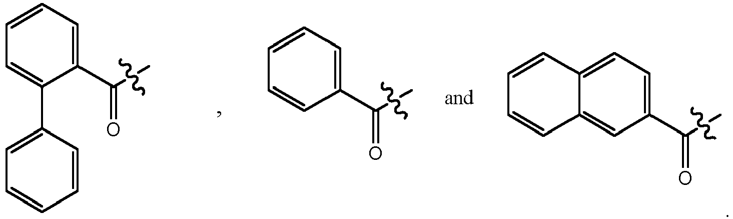

As another aspect of the present invention, the present invention may provide a pharmaceutical composition for preventing or treating cancer, including the compound represented by General Formula as an active ingredient.

As still another aspect of the present invention, the present invention provides a compound of the present invention for use in the prevention or treatment of cancer.

As used herein, the term "prevention" refers to all actions that block, suppress or delay symptoms caused by cancer by administering the composition according to the present invention.

As used herein, the term "treatment" refers to all actions that ameliorate or beneficially change symptoms caused by cancer by administering the composition according to the present invention.

As used herein, the term "subject" refers to a subject in need of prevention or treatment of a disease. For example, the subject may be a human or a mammal, including a non-human primate, a mouse, a dog, a cat, a horse, a sheep and a cow.

As used herein, the term "oligopeptide" refers to a linear molecule formed by bonding amino acid residues to each other by peptide bonds. The oligopeptide of the present invention may be prepared by a chemical synthesis method known in the art (for example, solid-phase synthesis techniques) along with a molecular biological method (Merrifield, J. Amer. Chem. Soc. 85: 2149-54(1963); Stewart, et al., Solid Phase Peptide Synthesis, 2nd. ed., Pierce Chem. Co.: Rockford, 111(1984)).

The scope of the compound according to the present invention may also include pharmaceutically acceptable salts thereof. As used herein, the term "pharmaceutically acceptable" refers to a compound which is suitable for use in contact with tissues of a subject (for example: a human) and within the scope of the sound medical judgment because its benefit/risk ratio is reasonable without excessive toxicity, irritation, allergic reactions or other problems or complications. The pharmaceutically acceptable salt includes, for example, acid addition salts formed by pharmaceutically acceptable free acids and pharmaceutically acceptable metal salts.

The pharmaceutical composition of the present invention is used for the prevention or treatment of cancer. Cancers for which the pharmaceutical composition of the present invention can be used may be a cancer selected from the group consisting of lung cancer, breast cancer, blood cancer, colorectal cancer, pancreatic cancer, and combinations thereof, but is not limited thereto.

In the present invention, the lung cancer may be non-small cell lung cancer, but is not limited thereto. In an exemplary embodiment of the present invention, the compounds may be for the treatment of lung cancer in T790M mutation-positive patients, EGFR m⁻ patients, and/or osimertinib resistant patients.

Moreover, the breast cancer may be Hormone receptor (HR) positive breast cancer, but is not limited thereto. Further, the breast cancer may be triple negative breast cancer, but is not limited thereto.

In addition, the blood cancer may be a blood cancer selected from the group consisting of leukemia, lymphoma, multiple myeloma, and combinations thereof, but is not limited thereto.

In an exemplary embodiment of the present invention, it was confirmed that the pharmaceutical composition according to the present invention exhibits excellent anticancer activity against lung cancer, breast cancer, blood cancer, pancreatic cancer and colorectal cancer (see Example 2).

In the present invention, when the pharmaceutical composition is used for preventing or treating lung cancer, the pharmaceutical composition includes a compound represented by General Formula X-AQTGTGKT as an active ingredient, and X is one or more selected from the group consisting of

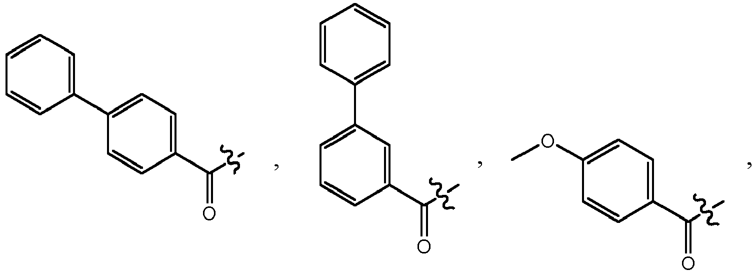

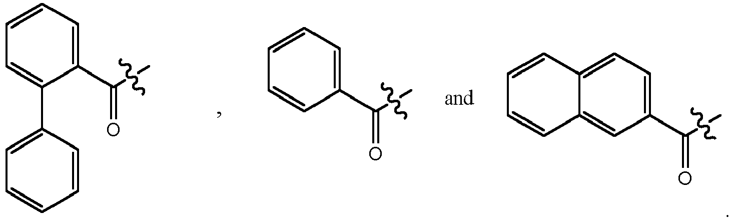

In addition, in the present invention, when the pharmaceutical composition is used for preventing or treating breast cancer, the pharmaceutical composition includes a compound represented by General Formula X-AQTGTGKT as an active ingredient, and X is one or more selected from the group consisting of

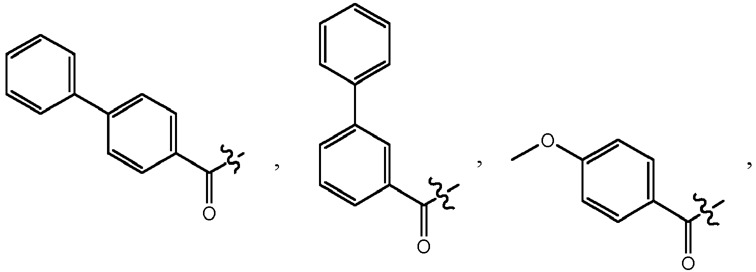

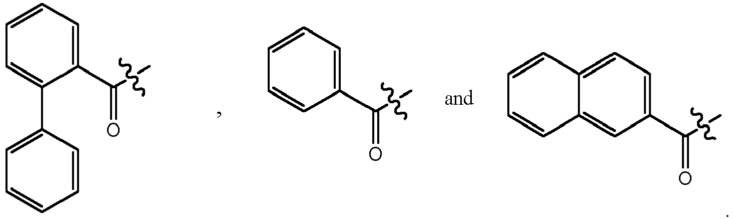

Furthermore, in the present invention, when the pharmaceutical composition is used for preventing or treating blood cancer, the pharmaceutical composition includes a compound represented by General Formula X-AQTGTGKT as an active ingredient, and X is one or more selected from the group consisting of

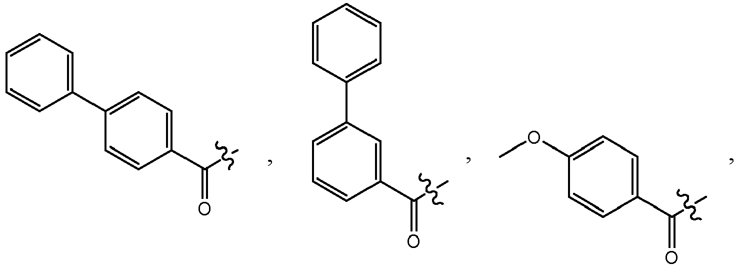

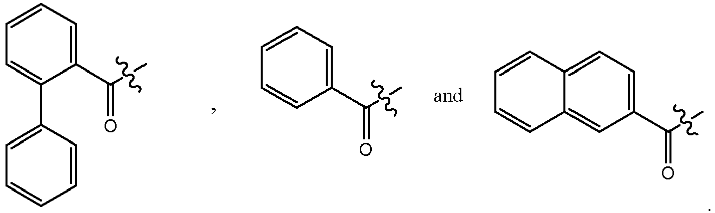

Further, in the present invention, when the pharmaceutical composition is used for preventing or treating pancreatic cancer, the pharmaceutical composition includes a compound represented by General Formula X-AQTGTGKT as an active ingredient, and X may be one or more selected from the group consisting of 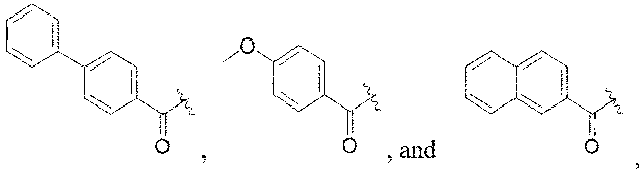 but is not limited thereto.

In addition, in the present invention, when the pharmaceutical composition is used for preventing or treating colorectal cancer, the pharmaceutical composition includes a compound represented by General Formula X-AQTGTGKT as an active ingredient, and X is one or more selected from the group consisting of

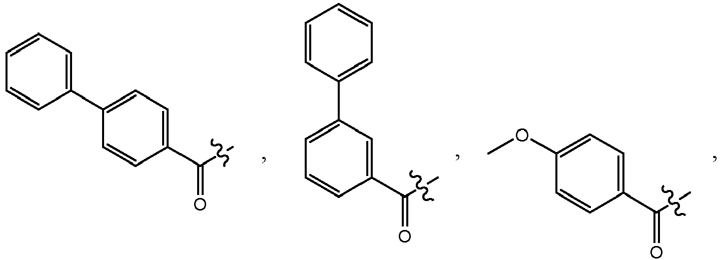

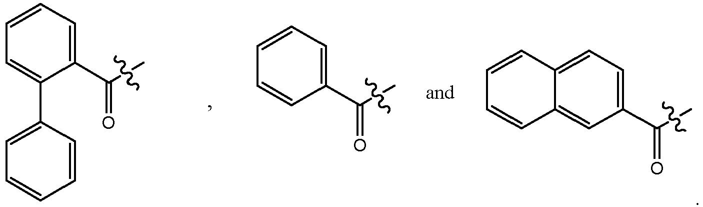

In another exemplary embodiment of the present invention, it was confirmed that the compounds represented by General Formula according to the present invention may be stably present in human blood, and have improved half-lives compared to AQTGTGKT (see Example 3). Therefore, the results of the Example show that the compounds according to the present invention have an improved anticancer effect and improved stability.

Further, in the compound represented by General Formula X-AQTGTGKT of the present invention, when X is one or more selected from the group consisting of 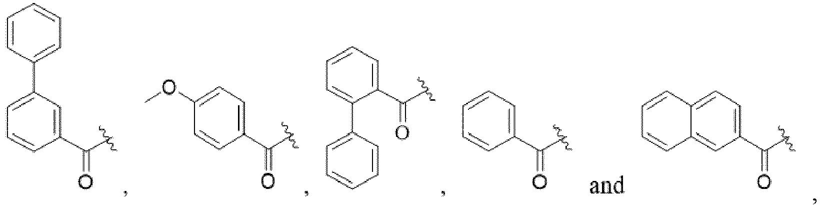 the half-life of the compound in human blood may be 45 minutes or more, but is not limited thereto. The half-life may be, for example, 45 minutes to 70 minutes, 46 minutes to 70 minutes, 47 minutes to 70 minutes, 48 minutes to 70 minutes, 49 minutes to 70 minutes, 50 minutes to 70 minutes, 51 minutes to 70 minutes, 53 minutes to 70 minutes, 55 minutes to 70 minutes, 57 minutes to 70 minutes, 59 minutes to 70 minutes, 65 minutes to 70 minutes, 45 minutes to 65 minutes, 50 minutes to 65 minutes, 55 minutes to 65 minutes, 60 minutes to 65 minutes, 45 minutes to 60 minutes, 50 minutes to 60 minutes, 55 minutes to 60 minutes, 45 minutes to 55 minutes, 50 minutes to 55 minutes, 60 minutes to 70 minutes, or the like. In addition, the half-life may be increased by 4400% to 6900% compared to the half-life of AQTGTGKT, but is not limited thereto. The increase rate may be, for example, 4400% to 6900%, 4500% to 6900%, 4600% to 6900%, 4700% to 6900%, 4800% to 6900%, 4900% to 6900%, 5000% to 6900%, 5100% to 6900%, 5200% to 6900%, 5300% to 6900%, 5400% to 6900%, 5500% to 6900%, 5600% to 6900%, 5700% to 6900%, 5800% to 6900%, 5900% to 6900%, 6000% to 6900%, 6100% to 6900%, 6200% to 6900%, 6300% to 6900%, 6400% to 6900%, 6500% to 6900%, 6600% to 6900%, 6700% to 6900%, 6800% to 6900%, 4400% to 6500%, 4500% to 6500%, 4700% to 6500%, 5000% to 6500%, 5200% to 6500%, 5500% to 6500%, 5700% to 6500%, 6000% to 6500%, 6300% to 6500%, 4400% to 6000%, 4600% to 6000%, 4800% to 6000%, 5000% to 6000%, 5500% to 6000%, 4400% to 5500%, 4700% to 5500%, 5000% to 5500%, 5200% to 5500%, 4400% to 5000%, 4800% to 5000%, 4400% to 4700%, 4500% to 4700%, or the like.

Furthermore, in the compound represented by General Formula X-AQTGTGKT of the present invention, when the X is 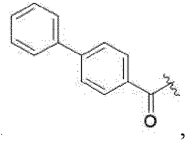 the half-life of the compound in human blood may be 100 minutes or more, but is not limited thereto. The half-life may be, for example, 100 minutes to 150 minutes, 102 minutes to 150 minutes, 103 minutes to 150 minutes, 104 minutes to 150 minutes, 105 minutes to 150 minutes, 106 minutes to 150 minutes, 107 minutes to 150 minutes, 108 minutes to 150 minutes, 109 minutes to 150 minutes, 110 minutes to 150 minutes, 111 minutes to 150 minutes, 112 minutes to 150 minutes, 115 minutes to 150 minutes, 117 minutes to 150 minutes, 120 minutes to 150 minutes, 123 minutes to 150 minutes, 125 minutes to 150 minutes, 127 minutes to 150 minutes, 130 minutes to 150 minutes, 132 minutes to 150 minutes, 135 minutes to 150 minutes, 137 minutes to 150 minutes, 140 minutes to 150 minutes, 142 minutes to 150 minutes, 145 minutes to 150 minutes, 147 minutes to 150 minutes, 148 minutes to 150 minutes, 100 minutes to 140 minutes, 102 minutes to 140 minutes, 104 minutes to 140 minutes, 106 minutes to 140 minutes, 108 minutes to 140 minutes, 120 minutes to 140 minutes, 125 minutes to 140 minutes, 130 minutes to 140 minutes, 135 minutes to 140 minutes, 100 minutes to 130 minutes, 102 minutes to 130 minutes, 105 to 130 minutes, 110 minutes to 130 minutes, 115 minutes to 130 minutes, 120 minutes to 130 minutes, 125 minutes to 130 minutes, 100 minutes to 120 minutes, 105 minutes to 120 minutes, 110 minutes to 120 minutes, 115 minutes to 120 minutes, 100 minutes to 110 minutes, 105 minutes to 110 minutes, 100 minutes to 105 minutes, or the like. Further, the half-life may be increased by 9900% to 14900% compared to the half-life of AQTGTGKT, but is not limited thereto. The increase rate may be, for example, 9900% to 14900%, 10000% to 14900%, 10100% to 14900%, 10200% to 14900%, 10300% to 14900%, 10400% to 14900%, 10500% to 14900%, 10700% to 14900%, 11000% to 14900%, 11500% to 14900%, 12000% to 14900%, 12500% to 14900%, 13000% to 14900%, 13500% to 14900%, 14000% to 14900%, 14500% to 14900%, 9900% to 12000%, 10000% to 12000%, 10200% to 12000%, 10500% to 12000%, 10700% to 12000%, 11000% to 12000%, 11500% to 12000%, 11700% to 12000%, 9900% to 11000%, 10000% to 11000%, 9900% to 10000%, or the like.

Meanwhile, the pharmaceutical composition according to the present invention may further include a suitable carrier, excipient and/or diluent which are/is typically used for preparation of a pharmaceutical composition in addition to the active ingredient. In addition, the pharmaceutical composition may be used by being formulated in the form of an oral formulation such as a powder, granules, a tablet, a capsule, a suspension, an emulsion, a syrup, and an aerosol, an external preparation, a suppository, and a sterile injection solution, according to a typical method.

Examples of the carrier, the excipient, and the diluent, which may be included in the composition include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, and the like. When the composition is prepared, the composition may be prepared using a commonly used diluent or excipient, such as a filler, an extender, a binder, a wetting agent, a disintegrant, and a surfactant.

The pharmaceutical composition according to the present invention is administered in a pharmaceutically effective amount. In the present invention, the "pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and other factors well known in the medical field. A preferred dosage of the preparation of the present invention may be selected depending on the condition and body weight of a subject, the degree of a disease, the form of drug, the administration route, and the duration. As a specific example, the pharmaceutical composition may be administered or injected in an amount of 0.001 to 1000 mg/kg, 0.01 to 100 mg/kg, 0.01 to 10 mg/kg, 0.1 to 10 mg/kg or 0.1 to 1 mg/kg once or several times per day.

It is important to administer the composition in a minimum amount that can obtain the maximum effect without any side effects, in consideration of all the aforementioned factors, and this amount may be determined by those skilled in the art. Specifically, the effective amount of the pharmaceutical composition according to the present invention may vary depending on the age, sex, condition, and body weight of a patient, the absorption rate, inactivation rate and excretion rate of the active ingredient *in vivo*, the type of the disease, and the drug to be used in combination.

The pharmaceutical composition of the present invention may be administered to an individual via various routes. For example, the pharmaceutical composition may be administered, for example, by oral administration, intranasal administration, transtracheal administration, arterial injection, intravenous injection, subcutaneous injection, intramuscular injection, or intraperitoneal injection. The daily dosage may be administered or injected once or in several divided doses per day.

As another aspect of the present invention, the present invention may provide a method for preparing an oligopeptide X-AQTGTGKT, the method including the following steps:
(A is alanine, Q is glutamine, T is threonine, G is glycine, and K is lysine, and
the X is one selected from the group consisting of 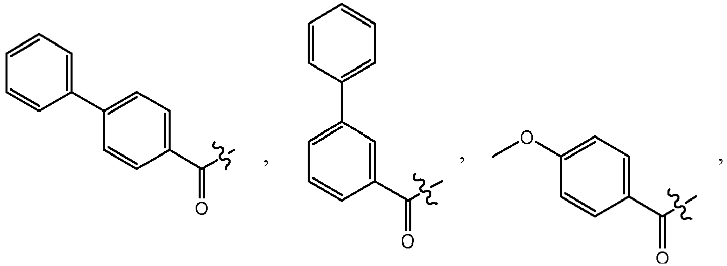 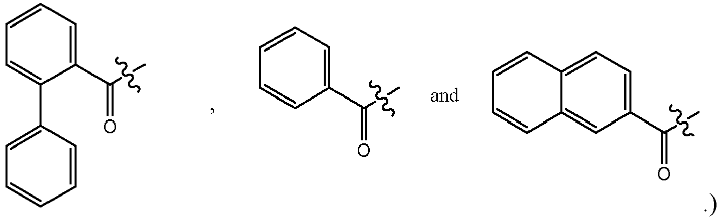
   (1) synthesizing each of TG and KT;
   (2) synthesizing TGKT by combining TG and KT;
   (3) synthesizing TGTGKT by bonding TG to the N-terminal of the TGKT;
   (4) synthesizing QTGTGKT by bonding Q to the N-terminal of the TGTGKT; and
   (5) synthesizing X-AQTGTGKT by bonding an alanine derivative (X-A) to the N-terminal of the QTGTGKT.

Hereinafter, preferred examples for helping the understanding of the present invention will be suggested. However, the following examples are provided only to more easily understand the present invention, and the contents of the present invention are not limited by the following examples. The following examples relate, in part, to a compound named Ac-AQTGTGKT. The compound Ac-AQTGTGKT is not a part of the present invention.

### [Examples]

### Experimental method

### 1. CellTiter-Glo Luminescent (CTG) assay

After cancer cell lines were treated with the AQTGTGKT analog compound according to the present invention, the degree of cell proliferation was measured by CTG assay. Specifically, cells were seeded on a 96-well plate at 5 × 10³ cells/100 µl per well, cultured for 24 hours, and then transfected with seven types of AQTGTGKT analogs according to the present invention. After 48 hours, a CellTiter-Glo^{®} (Promega Co., USA) reagent was mixed in the same amount as the cell culture medium and allowed to react in an orbital shaker for 2 minutes. After reaction at room temperature for 10 minutes, the luminescence signal was measured using a luminometer (GloMax^{®}, Promega).

### 2. Tetrazolium (MTT) assay

After cancer cell lines were treated with the AQTGTGKT analog compound according to the present invention, the degree of cell proliferation was measured by MTT assay. Specifically, cells were seeded on a 96-well plate at 5 × 10³ cells/100 µl per well, cultured for 24 hours, and then transfected with seven types of AQTGTGKT analogs according to the present invention. After 72 hours, 10 µl of a CellTiter-96^{®} (Promega Co., USA) reagent was added to each well and allowed to react at 5% CO₂ and 37°C. After 3 hours, absorbance was measured at 490 nm using a spectrophotometer (SPECTROstar^{Nano}, BMG).

### 3. Lung cancer animal model

150 µl of H1975 cells were inoculated once by subcutaneous injection into the flanks of mice at a concentration of 4×10⁶ cells/mouse. After it was confirmed that the volume of a tumor mass formed after the inoculation reached 70 to 130 mm³, random group separation was performed. A test material was injected to the tail vein 5 times at 3-day intervals and 5 times at 2-day intervals.

200 µl of H820 cells were inoculated once by subcutaneous injection into the flanks of mice at a concentration of 5×10⁶ cells/mouse at 1:1 with Matrigel. After it was confirmed that the volume of a tumor mass formed after the inoculation reached 70 to 130 mm³, random group separation was performed. The test material was injected to the tail vein 14 times daily.

### 4. Breast cancer animal model

200 µl of a breast cancer cell line, HCC1806 cells, were inoculated once by subcutaneous injection into the flanks of mice at a concentration of 5×10⁶ cells/mouse. After it was confirmed that the volume of a tumor mass formed after the inoculation reached 70 to 130 mm³, random group separation was performed. A test material was injected to the tail vein 7 times at 2-day intervals at a concentration of 10 mpk, and the start date of administration was set as day 1. The tumor volume was calculated twice a week by measuring the width and length and at the same time, the mouse body weight was measured.

### 5. Colorectal cancer animal model

200 µl of CT26 cells, which were induced to express *CAGE* gene, were inoculated once by subcutaneous injection into the flanks of mice at a concentration of 1×10⁶ cells/mouse. After it was confirmed that the volume of a tumor mass formed after the inoculation reached 70 mm³, random group separation was performed. A test material was injected to the tail vein 7 times at 2-day intervals at a concentration of 10 mpk, and the start date of administration was set as day 1. The tumor volume was calculated twice a week by measuring the width and length and at the same time, the mouse body weight was measured.

### Example 1: Preparation of AQTGTGKT analog

### 1.1. General reactions

All reactions were performed using commercially available materials and reagents without additional reactions, unless otherwise stated. The reactions were monitored by thin film chromatography (TLC) on silica gel plates (Keiselgel 60 F254, Merck) and/or ultra-high performance liquid chromatography (UPLC). The spots on the TLC plate was visualized by staining the TLC plate with UV light and with potassium permanganate and/or carbonizing the TLC plate with a heat gun. All products were characterized using ¹H NMR and/or UPLC-MS.

### 1.2. Synthesis of Boc/OBn-TG

First, TG whose functional group was protected with benzyl was synthesized according to the following Reaction Scheme 1. Hereinafter, the compound in each reaction scheme will be referred to as Compound n according to the Arabic numeral (n) described below.

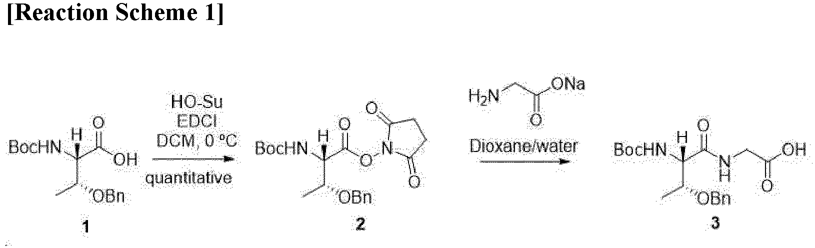

Specifically, BocThr(OBn)OH (Compound 1; 25.0 g, 80.8 mmol, and 1.0 eq) and NOSu (9.77 g, 84.8 mmol, and 1.05 eq) were dissolved in dichloromethane (150 mL). The mixture was cooled to 0°C and placed in an inert atmosphere. Then, 1-(3-dimethylaminopropyl)-3-ethylcarbodiimide hydrochloride (16.3 g, 84.8 mmol, and 1.05 eq) was added to the mixture. The mixture was warmed to room temperature and stirred for 20 hours. Subsequently, the mixture was washed with NH₄Cl (sat. aq.) and phases were separated. The organic layer was dried over MgSO₄ and concentrated under reduced pressure to obtain Compound 2 as a pale yellow oil (35.7 g, >100% yield, assuming a quantitative yield) as a product.

Compound 2 BocThr(OBn)OSu (32.8 g, 80.8 mmol, and 1.0 eq) was dissolved in 1,4-dioxane (200 mL), and a solution of glycine sodium salt hydrate in distilled water (100 mL) was added thereto in one portion. After being stirred at room temperature for 6 hours, the mixture was fractionated into ethyl acetate and citric acid (sat. aq.). The organic layer was dried over MgSO₄, filtered, and then concentrated under reduced pressure. The crude material was purified by 30 to 70% acetonitrile (0.1% formic acid) in a water (0.1% formic acid) eluent in a C18 (400 g) column. The desired fractions were combined and fractionated into ethyl acetate and NaHCO₃ (sat. aq.). After the organic layer was dried over MgSO₄ and filtered, Compound 3 as a pale yellow gum (21.9 g, a yield of 74%) as a product was obtained under reduced pressure.

### 1.3. Synthesis of CBz/OBn/CO₂Bn-KT

KT whose OH functional group was protected with benzyl was synthesized according to the following Reaction Scheme 2.

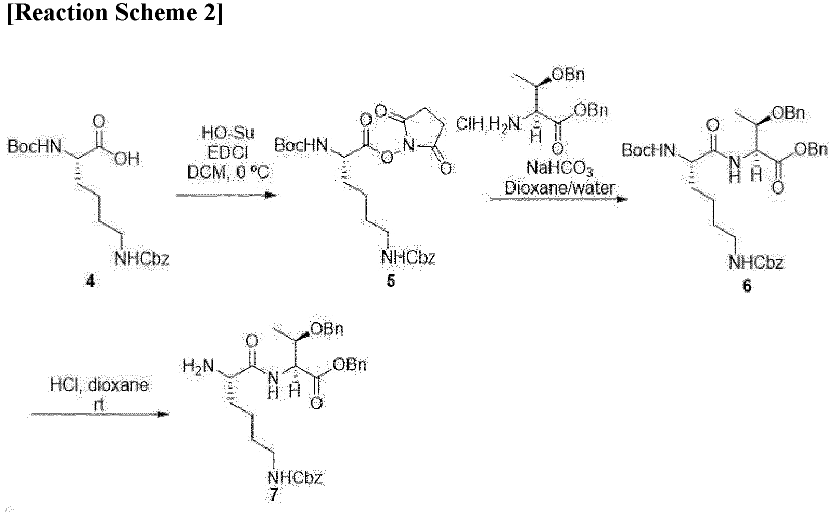

Specifically, BocLys(CBz)OH (Compound 4; 27.0 g, 70.9 mmol, and 1.0 eq) and NOSu (9.80 g, 85.1 mmol, and 1.2 eq) were dissolved in dichloromethane (128 mL). The mixture was cooled to 0°C and placed in an inert atmosphere. Then, 1-(3-dimethylaminopropyl)-3-ethylcarbodiimide hydrochloride (16.3 g, 85.1 mmol, and 1.05 eq) was added to the mixture. The mixture was warmed to room temperature and stirred for 20 hours. Subsequently, the mixture was washed with NH₄Cl (sat. aq.) and phases were separated. The organic layer was dried over MgSO₄ and concentrated under reduced pressure to obtain Compound 5 as a pale yellow oil (36.7 g, >100% yield, assuming a quantitative yield) as a product.

Subsequently, Compound 5 (BocLys(Cbz)OSu; 36.7 g, 70.9 mmol, and 1.0 eq) and Thr(OBn)OBn.HCl (25.0 g, 74.4 mmol, and 1.05 eq) were dissolved in 1,4-dioxane (477 mL) at room temperature. A solution of NaHCO₃ (6.85 g, 81.5 mmol, and 1.15 eq) in distilled water (326 mL) was added to the above solution. Then, the produced mixture was stirred at room temperature for 20 hours. The reaction mixture was diluted with ethyl acetate and washed with 10% citric acid (aqueous) and brine. The organic layer was dried over Na₂SO₄, filtered under reduced pressure, and concentrated under reduced pressure to obtain 55.9 g (>100% yield, assuming a quantitative yield) of Compound 6 as a yellow oily solid as a product.

Finally, Compound 6 (BocLys(Cbz)Thr(OBn)OBn; 55.9 g, 70.9 mmol, and 1.00 eq) was dissolved in 1,4-dioxane (360 mL), and 4 N HCl in 1,4-dioxane (177 mL) was added thereto. The mixture was stirred at room temperature overnight. Then, a saturated aqueous solution of NaHCO₃ was added thereto until the pH value reached 8. The solution was extracted with ethyl acetate, and the produced organic solution was dried over Na₂SO₄, filtered, and then concentrated under reduced pressure to obtain Compound 7 as a yellow oily solid (38.7 g, 97% yield) as a product.

### 1.4. Synthesis of CBz/OBn/OBn/CO₂Bn-TGKT

TGKT was synthesized by combining TG and KT synthesized in 1.2. and 1.3. according to the following Reaction Scheme 3.

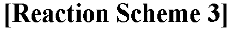

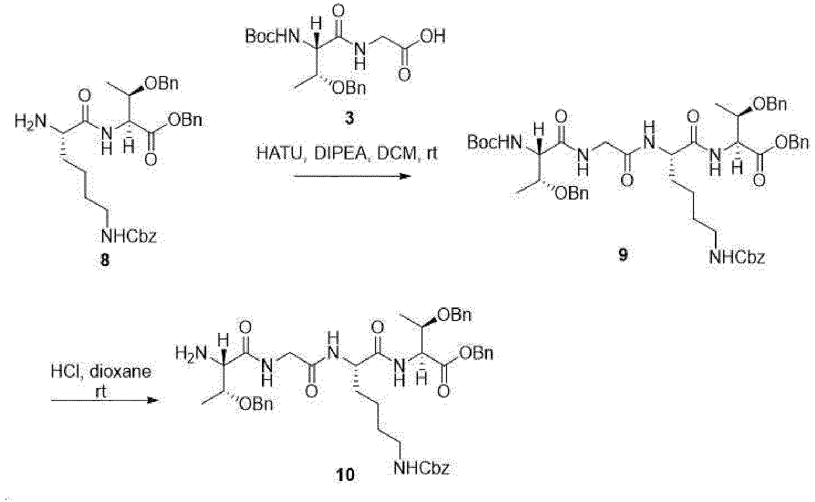

More specifically, N,N-diisopropylethylamine was added to a solution of BocThr(OBn)GlyOH (Compound 3; 5.61 g, 15.3 mmol, and 1.00 eq) and Compound 8 (Lys(Cbz)Thr(OBn)OBn; 10.0 g, 15.3 mmol, and 1.0 eq) in dichloromethane (50 mL). The mixture was stirred at room temperature in an inert atmosphere, and HATU (7.00 g, 18.4 mmol, and 1.20 eq) was added thereto. The produced mixture was stirred for 2 hours, washed with NH₄Cl (sat. aq.), and subsequently washed with NaHCO₃ (sat. aq.). The organic layer was dried over Na₂SO₄, filtered, and then concentrated under reduced pressure to obtain Compound 9 as a pale orange oily solid (25.0 g, >100% yield, and assuming a quantitative yield) as a product.

The obtained BocThr(OBn)GlyLys(Cbz)Thr(OBn)OBn (Compound 9; 13.9 g taken in the previous step, 15.3 mmol, and 1.0 eq) was dissolved in 1,4-dioxane (150 mL) at room temperature under nitrogen. 4 N HCl in 1,4-dioxane (20 mL) was added to the solution. The mixture was stirred at room temperature for 20 hours. The mixture was concentrated under reduced pressure and purified in a C18 (400 g) column using 20% acetonitrile (0.1% formic acid) in a water (0.1% formic acid) eluent. The desired fractions were combined and lyophilized. The produced powder was dissolved in NaHCO₃ (sat. aq.) and dichloromethane, and the resulting solution was stirred for 15 minutes. Layers were separated, and the organic layer was dried over Na₂SO₄, filtered, and then concentrated under reduced pressure to obtain Compound 10 as a colorless gum (10.9 g, 88% yield) as a product.

### 1.5. Synthesis of CBz/OBn/OBn/OBn/CO₂Bn-TGTGKT

TGTGKT protected with benzyl was synthesized by combining TG (Compound 3) and TGKT (Compound 10) synthesized in 1.2. and 1.4. according to the following Reaction Scheme 4.

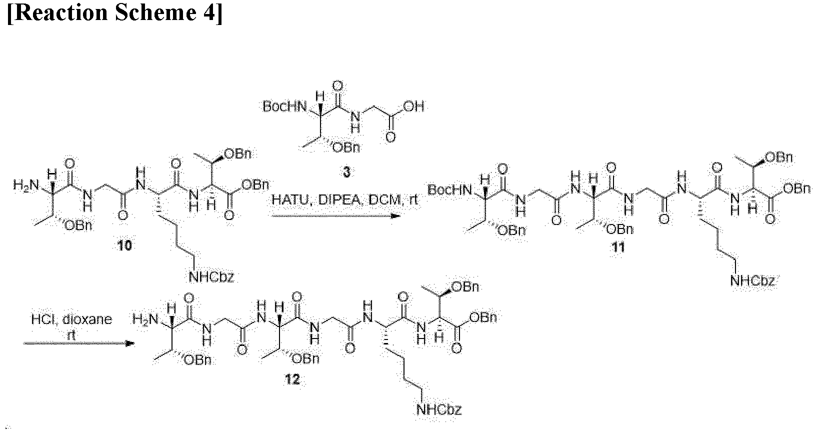

More specifically, *N,N*-diisopropylethylamine (5.10 mL, 29.3 mmol, and 2.2 eq) was added to a solution of BocThr(OBn)GlyOH (Compound 3; 5.10 g, 14.0 mmol, and 1.05 eq) and BocThr(OBn)GlyLys(Cbz)Thr(OBn) OBn (Compound 10; 10.8 g, 13.3 mmol, and 1.0 eq) in dichloromethane (100 mL). The mixture was stirred at room temperature in an inert atmosphere, and HATU (5.60 g, 14.7 mmol, and 1.1 eq) was added thereto. The produced mixture was stirred for 2 hours, and washed with NH₄Cl (sat. aq.) and NaHCO₃ (sat. aq.). The organic layer was concentrated under reduced pressure to obtain Compound 11 as a pale yellow gum (21.4 g, >100% yield, assuming a quantitative yield) as a product.

Subsequently, the obtained Compound 11 (BocThr(OBn)GlyThr(OBn)GlyLys(Cbz)Thr (OBn)OBn; 15.4 g taken in the previous step, 13.3mmol, and 1.00 eq) was dissolved in 1,4-dioxane (150 mL) at room temperature under nitrogen. 4 N HCl in 1,4-dioxane (50 mL) was added to the solution, and the mixture was stirred at room temperature for 5 hours. The mixture was concentrated under reduced pressure and purified in a C18 (120 g) column using 20% acetonitrile (0.1% formic acid) in a water (0.1% formic acid) eluent. The desired fractions were combined, concentrated to half of the volume, and then fractionated into NaHCO₃ (sat. aq.) and ethyl acetate. Layers were separated, and the organic layer was dried over Na₂SO₄, filtered, and concentrated under reduced pressure to obtain Compound 12 as an ash-colored solid (14.6 g, >100% yield, and assuming a quantitative yield) as a product.

### 1.6. Synthesis of CBz/OBn/OBn/OBn/CO₂Bn-QTGTGKT

Compound 14 (QTGTGKT) was synthesized by additionally combining Q with Compound 12 (TGTGKT) synthesized in 1.5. according to the following Reaction Scheme 5.

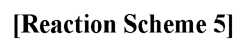

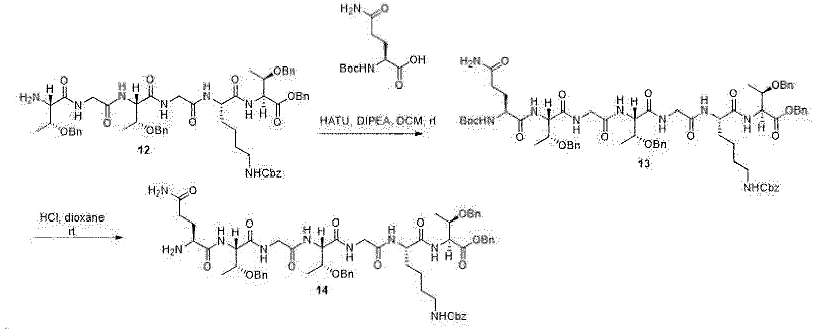

More specifically, N,N-diisopropylethylamine (4.60 mL, 26.4 mmol, and 2.2 eq) was added to a solution of Thr(OBn)GlyThr(OBn)GlyLys(Cbz)Thr(OBn)OBn (Compound 12; 12.7 g, 12.0 mmol, and 1.0 eq) and BocGlnOH (3.25 g, 13.2 mmol, and 1.1 eq) in ethyl acetate (150 mL) and *N,N*-dimethylformamide (25 mL). The mixture was stirred at room temperature in an inert atmosphere, and HATU (5.47 g, 14.4 mmol, and 1.20 eq) was added thereto. The produced mixture was stirred for 1 hour, and then washed with NH₄Cl (sat. aq.). The organic layer was additionally extracted with dichloromethane. Subsequently, the organic layer was combined and concentrated under reduced pressure. The crude material was purified in a 400 g C18 column using a 20-100% acetonitrile (0.1% formic acid) gradient in water (0.1% formic acid). The desired fractions were combined, and then fractionated into a solution of ethyl acetate and NaHCO₃ (sat. aq.). The organic layer was concentrated, and residual water was removed by a lyophilization process. A total of 12.9 g (89% total yield) of Compound 13 was obtained from the combined fractions.

The obtained Compound 13 (BocGlnThr(OBn)GlyThr(OBn)GlyLys(Cbz)Thr(OBn) OBn; 7.00 g, 5.40 mmol, and 1.00 eq) was dissolved in 1,4-dioxane (150 mL) at room temperature under nitrogen. 4 N HCl in 1,4-dioxane (43.5 mL) was added to the solution. The mixture was stirred at room temperature for 20 hours. The mixture was concentrated under reduced pressure and lyophilized using a water/acetonitrile (2/1) solution. Finally, Compound 14 as a pale yellow powder (6.36 g, 96% yield) was separated.

### 1.7. Synthesis of AQTGTGKT analog

The remaining 6 types of analogs except for 4-PhPh-AQTGTGKT were synthesized by combining Compound 14 which is a final product of Reaction Scheme 5 and Compound 17n which is a product of the following Reaction Scheme 6 according to the following Reaction Scheme 7.

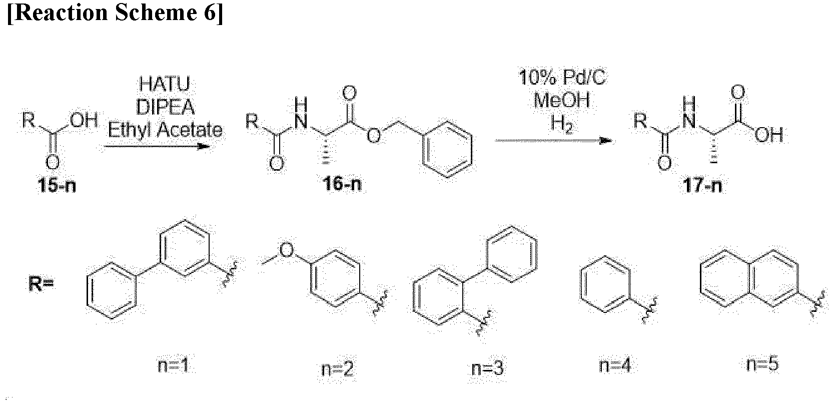

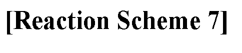

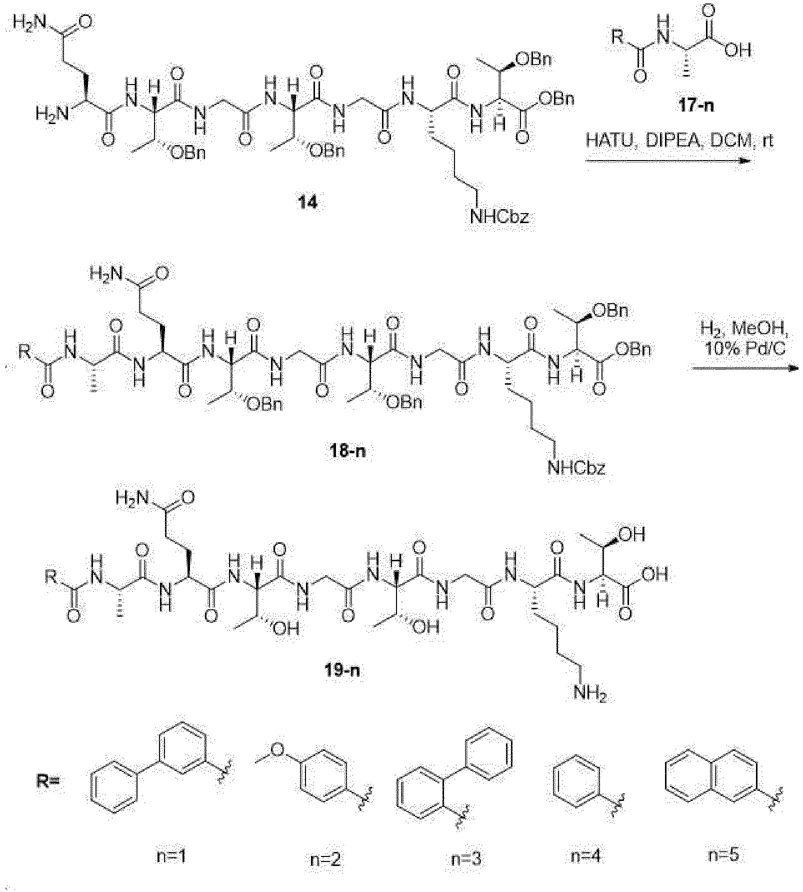

According to Reaction Scheme 7, 2.9 mg of 3-PhPh-AQTGTGKT with a purity of 90% or more, 7.0 mg of 4-MeOPh-AQTGTGKT with a purity of 89%, 22.7 mg of 2-PhPh-AQTGTGKT with a purity of 95% or more, 23.2 mg of Ph-AQTGTGKT with a purity of 90% or more, and 23.2 mg of Naphthyl-AQTGTGKT with a purity of 85% were finally obtained.

Hereinafter, each analog synthesis process will be specifically described.

### 1.7.1. Synthesis of 3-PhPh-AQTGTGKT

For 3-PhPh-AQTGTGKT (Compound 19-1), 3-PhPh-AQTGTGKT, which is a final target compound, was synthesized by reacting Compound 17-1 obtained by the following Reaction Scheme 8 with Compound 14 according to Reaction Scheme 9.

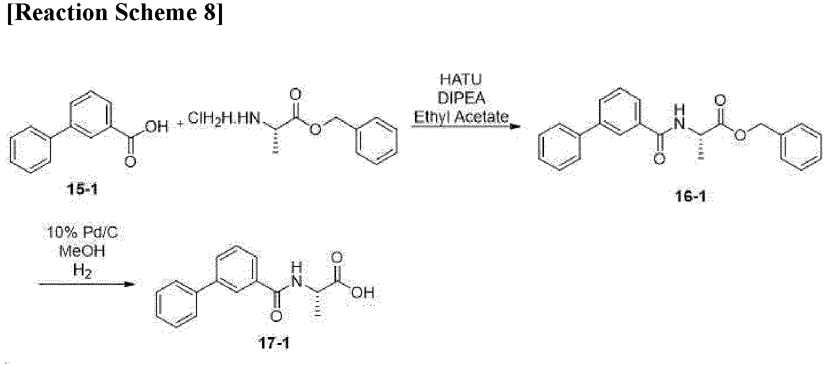

More specifically, H-Ala-OBzl.HCl (388 mg, 1.80 mmol, and 1.2 eq) was suspended in ethyl acetate (10 mL), and N,N-diisopropylethylamine (653 µL, 3.75 mmol, and 2.5 eq) was added thereto. After the resulting mixture was stirred at room temperature for 5 minutes, HATU (855 mg, 2.25 mmol, and 1.5 eq) and [1,1'-biphenyl]-3-carboxylic acid (297 mg, 1.50 mmol, and 1 eq) were added thereto, and the mixture was stirred at room temperature for 2 hours. The reaction mixture was diluted with ethyl acetate, and then washed with NH₄Cl (sat. aq.), NaHCO3 (sat. aq.) and brine. The obtained organic material was dried (Na₂SO₄), filtered, and then concentrated under reduced pressure. The residue was purified by a 2-40% ethyl acetate gradient in heptane in a 25 g column to obtain Compound 16-1 as a colorless solid (493 mg, 91% yield).

Subsequently, 10% Pd/C (49 mg) soaked with a minimum amount of water was added to a solution of Compound 16-1 (3-PhPh-AlaOBn; 493 mg, 1.37 mmol) in methanol (30 mL). The mixture was stirred under a hydrogen atmosphere (balloon) for 2 hours. The mixture was filtered through a Celite pad and washed with methanol and ethyl acetate. The obtained filtrate was concentrated under reduced pressure to obtain Compound 17-1 as a colorless foam (337 mg, 91% yield), which was reacted with Compound 14 according to the following Reaction Scheme 9.

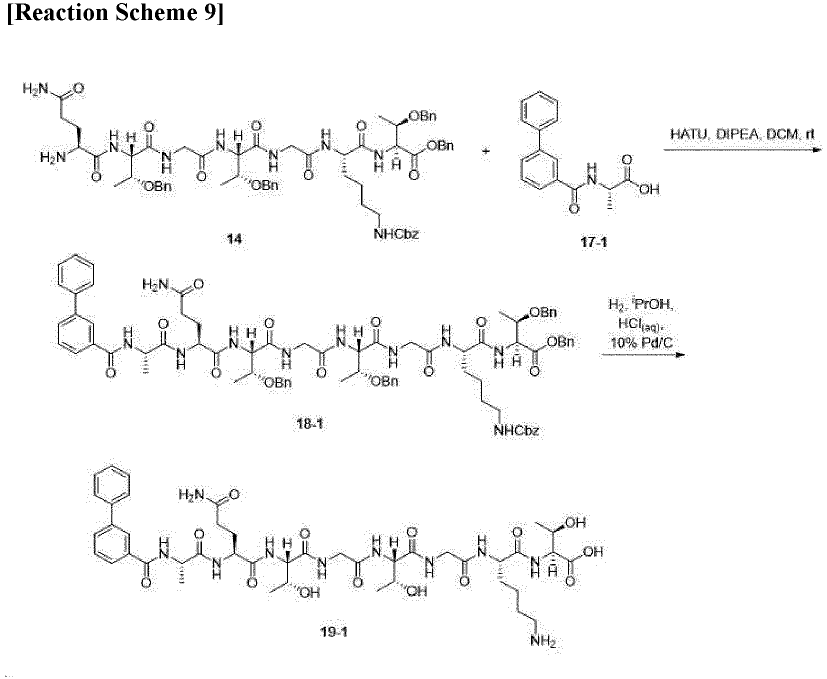

More specifically, HATU (106 mg, 0.278 mmol, and 1.1 eq) was added to a suspension of GlnThr(OBn)GlyThr(OBn)GlyLys(Cbz) Thr(OBn)OBn (Compound 14; 300 mg, 0.253 mmol, and 1 eq) and 3-PhPh-AlaOH (Compound 17-1; 68.0 mg, 0.253 mmol, and 1 eq) in *N, N*-diisopropylethylamine (97.0 µL, 0.556 mmol, and 2.2 eq) and dichloromethane (20 mL). The mixture was stirred at room temperature for 2 hours, and the reaction mixture was washed with NaHCO₃ (sat. aq.). The organic material was concentrated under reduced pressure, the residue was purified by a 40-100% acetonitrile (0.1% formic acid) gradient in water (0.1% formic acid) in a 60 g C18 column and lyophilized, and then Compound 18-1 as a colorless solid (140 mg, 38% yield) was obtained.

Subsequently, 10% Pd/C (85.0 mg) was added to a solution of 3-PhPh-AlaGlnThr(OBn)GlyThr(OBn)GlyLys(Cbz)Thr(OBn)OBn (Compound 18-1; 85.0 mg, 59.1 µmol) in 2 M hydrochloric acid (aqueous, 0.5 mL) and 2-propanol (10 mL). After the mixture was stirred under a hydrogen atmosphere (balloon) for 4.5 hours, the mixture was filtered through a 0.45 µm syringe filter. The obtained filtrate was concentrated under reduced pressure, and the residue was lyophilized. Then, the material was purified in a 60 g C18 column using 5-50% acetonitrile (0.1% formic acid) in water (0.1% formic acid), and lyophilized to obtain a target Compound 19-1 as a colorless solid (2.9 mg, 5% yield).

¹H NMR data of Compound 19-1 (3-PhPh-AQTGTGKT) was measured as follows:
¹H NMR (400 MHz; D2O): δ = 8.01-7.99 (m, 1H), 7.86-7.82 (m, 1H), 7.75-7.66 (m, 3H), 7.55 (t, J 7.7 Hz, 1H), 7.49 (t, J 7.5 Hz, 2H), 7.43-7.38 (m, 1H), 4.48-4.24 (m, 5H), 4.23-4.12 (m, 3H), 4.09 (d, J 3.8 Hz, 1H), 4.00- 3.96 (m, 2H), 3.88 (s, 2H), 2.90 (t, J 7.4Hz, 2H), 2.35 (t, J 7.5Hz, 2H), 2.15-2.06 (m, 1H), 2.03-1.91 (m , 1H), 1.84-1.72 (m, 1H), 1.70-1.52 (m, 3H), 1.45 (d, J 7.2Hz, 3H), 1.40-1.24 (m, 2H), 1.15-1.05 (m, 9H), 16 exchangeable protons not visible.

### 1.7.2. Synthesis of 4-MeOPh-AQTGTGKT

For 4-MeOPh-AQTGTGKT (Compound 19-2), 4-MeOPh-AQTGTGKT, which is a final target compound, was synthesized by reacting Compound 17-2 obtained by the following Reaction Scheme 10 with Compound 14 according to Reaction Scheme 11.

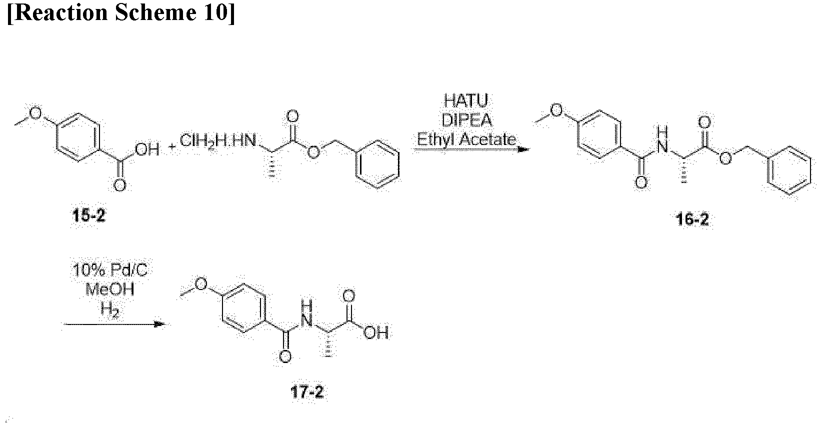

More specifically, H-Ala-OBzl.HCl (425 mg, 1.97 mmol, and 1.2 eq) was suspended in ethyl acetate (10 mL), and *N,N*-diisopropylethylamine (715 µL, 4.11 mmol, and 2.5 eq) was added thereto. After the resulting mixture was stirred at room temperature for 5 minutes, HATU (937 mg, 2.46 mmol, and 1.5 eq) and 4-methoxybenzoic acid (250 mg, 1.64 mmol, and 1 eq) were added thereto, and the mixture was stirred at room temperature for 2 hours. The reaction mixture was diluted with ethyl acetate, and then washed with NH₄Cl (sat. aq.), NaHCO₃ (sat. aq.) and brine. The organic material was dried (Na₂SO₄), filtered, and then concentrated under reduced pressure. The residue was purified by a 15-50% ethyl acetate gradient in heptane in a 25 g column to obtain Compound 16-2 as a colorless solid (360 mg, 70% yield).

Subsequently, 10% Pd/C (18 mg) soaked with a minimum amount of water was added to a solution of 4-OMePh-AlaOBn (Compound 16-2; 180 mg, 0.574 mmol) in methanol (15 mL). The mixture was stirred under a hydrogen atmosphere (balloon) for 110 hours. Then, the mixture was filtered through a Celite pad and washed with methanol. The obtained filtrate was concentrated under reduced pressure to obtain Compound 17-2 as a colorless oil (128 mg, 100% yield), which was reacted with Compound 14 according to the following Reaction Scheme 11.

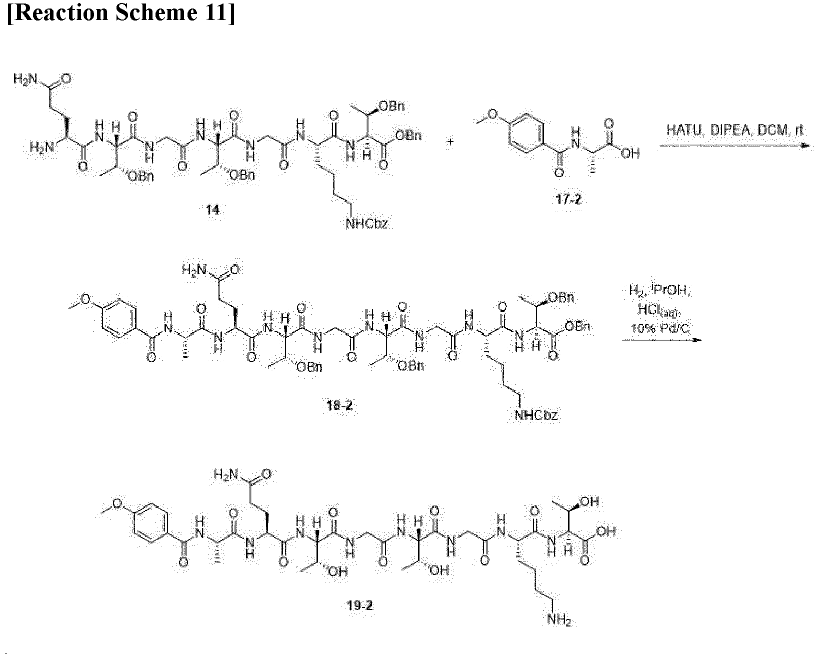

More specifically, HATU (74.5 mg, 0.196 mmol, and 1.2 eq) was added to a suspension of GlnThr(OBn)GlyThr(OBn)GlyLys (Cbz)Thr(OBn)OBn (Compound 14; 200 mg, 0.164 mmol, and 1 eq) and 4-OMePh-AlaOH (Compound 17-2; 36.6 mg, 0.164 mmol, and 1 eq) in *N, N*-diisopropylethylamine (63.0 µL, 0.360 mmol, and 2.2 eq) and dichloromethane (20 mL). The mixture was stirred at room temperature for 64 hours, and the reaction mixture was diluted with methanol, and then washed with NH₄Cl (sat. aq.), NaHCO₃ (sat. aq.) and water. The organic material was concentrated under reduced pressure, the residue was purified by a 50-95% acetonitrile (0.1% formic acid) gradient in water (0.1% formic acid) in a 60 g C18 column and lyophilized, and then Compound 18-2 as a colorless solid (113 mg, 50% yield) was obtained.

Subsequently, 10% Pd/C (100 mg) was added to a solution of 4-OMePh-AlaGlnThr(OBn)GlyThr(OBn)GlyLys(Cbz)Thr(OBn)OBn (Compound 18-2; 108 mg, 77.6 µmol) in 2 M hydrochloric acid (aqueous, 1.0 mL) and 2-propanol (20 mL). The mixture was stirred under a hydrogen atmosphere (balloon) for 18 hours. The mixture was filtered through a 0.45 µm syringe filter. The obtained filtrate was concentrated under reduced pressure, and the residue was dissolved in water, and then lyophilized. The dried material was purified in a 30 g C18 column using a 5-30% acetonitrile (0.1% formic acid) gradient in water (0.1% formic acid) and lyophilized to obtain Compound 19-2 as a colorless solid (7.0 mg, 10% yield).

¹H NMR data of Compound 19-2 (4-MeOPh-AQTGTGKT) was measured as follows:
¹H NMR (400 MHz; D2O): δ = 7.76-7.71 (m, 2H), 7.02-6.98 (m, 2H), 4.42-4.28 (m, 5H), 4.24-4.13 (m, 3H), 4.09 (d, J 3.9 Hz, 1H), 4.01-3.96 (m, 2H), 3.89 (s, 2H), 3.81 (s, 3H), 2.91 (t, J 7.4 Hz, 2H), 2.34 (t, J 7.6 Hz, 2H), 2.14-2.06 (m, 1H), 2.00-1.91 (m, 1H), 1.85-1.75 (m, 1H), 1.71-1.54 (m, 3H), 1.42 (d, J 7.2 Hz, 3H), 1.40-1.25 (m, 3H), 1.16-1.07 (m, 8H), 16 exchangeable protons not visible.

### 1.7.3. Synthesis of 2-PhPh-AQTGTGKT

For 2-PhPh-AQTGTGKT (Compound 19-3), 2-PhPh-AQTGTGKT, which is a final target compound, was synthesized by reacting Compound 17-3 obtained by the following Reaction Scheme 12 with Compound 14 according to Reaction Scheme 13.

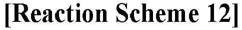

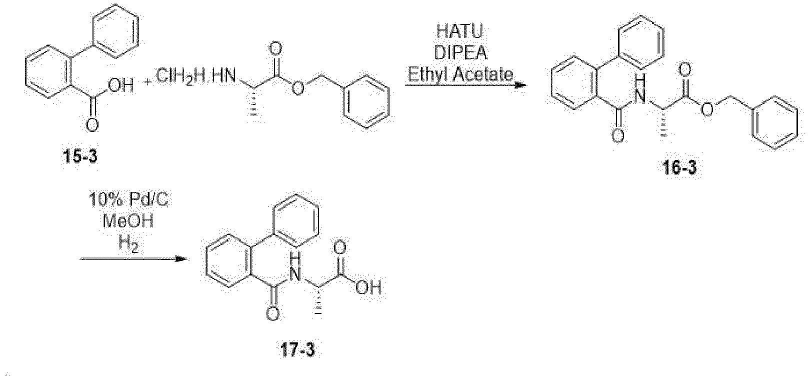

More specifically, H-Ala-OBzl.HCl (388 mg, 1.80 mmol, and 1.2 eq) was suspended in ethyl acetate (10 mL), and *N,N*-diisopropylethylamine (653 µL, 3.75 mmol, and 2.5 eq) was added thereto. After the resulting mixture was stirred at room temperature for 5 minutes, HATU (855 mg, 2.25 mmol, and 1.5 eq) and [1,1'-biphenyl]-2-carboxylic acid (297 mg, 1.50 mmol, and 1 eq) were added thereto, and the mixture was stirred at room temperature for 2 hours. The reaction mixture was diluted with ethyl acetate, and then washed with NH₄Cl (sat. aq.), NaHCO₃ (sat. aq.) and brine. The obtained organic material was dried (Na₂SO₄), filtered, and then concentrated under reduced pressure. The residue was purified by a 2-40% ethyl acetate gradient in heptane in a 25 g column to obtain Compound 16-3 as a colorless oil (416 mg, 77% yield).

Subsequently, 10% Pd/C (42 mg) soaked with a minimum amount of water was added to a solution of 2-PhPh-AlaOBn (Compound 16-3; 416 mg, 1.16 mmol) in methanol (20 mL). The mixture was stirred under a hydrogen atmosphere (balloon) for 18 hours. Then, the mixture was filtered through a Celite pad and washed with methanol. The obtained filtrate was concentrated under reduced pressure to obtain Compound 17-3 as a colorless oil (308 mg, 99% yield), which was reacted with Compound 14 according to the following Reaction Scheme 13.

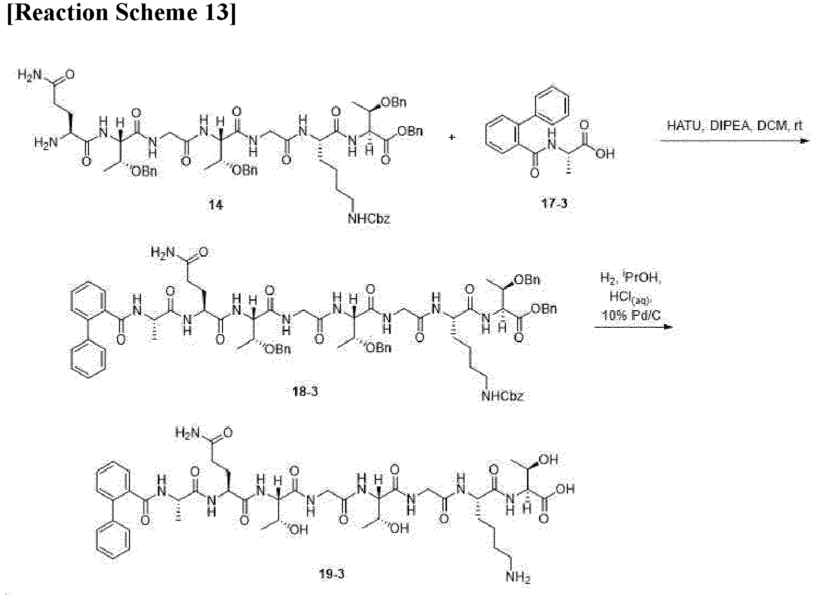

More specifically, HATU (74.5 mg, 0.196 mmol, and 1.2 eq) was added to a suspension of GlnThr(OBn)GlyThr(OBn)GlyLys(Cbz) Thr(OBn)OBn (Compound 14; 200 mg, 0.164 mmol, and 1 eq) and 2-PhPh-AlaOH (Compound 17-3; 44.2 mg, 0.164 mmol, and 1 eq) in *N,N*-diisopropylethylamine (63.0 µL, 0.360 mmol, and 2.2 eq) and dichloromethane (20 mL). The mixture was stirred at room temperature for 64 hours, and the reaction mixture was diluted with methanol, and then washed with NH₄Cl (sat. aq.), NaHCO₃ (sat. aq.) and water. The organic material was concentrated under reduced pressure, the residue was purified by a 50-95% acetonitrile (0.1% formic acid) gradient in water (0.1% formic acid) in a 60 g C18 column and lyophilized, and then Compound 18-3 as a colorless solid (115 mg, 49% yield) was obtained.

Subsequently, 10% Pd/C (100 mg) was added to a solution of 2-PhPh-AlaGlnThr(OBn)GlyThr(OBn)GlyLys(Cbz)Thr(OBn)OBn (Compound 18-3; 110 mg, 76.5 µmol) in 2 M hydrochloric acid (aqueous, 1.0 mL) and 2-propanol (20 mL). The mixture was stirred under a hydrogen atmosphere (balloon) for 18 hours. The mixture was filtered through a 0.45 µm syringe filter. The obtained filtrate was concentrated under reduced pressure, and the residue was dissolved in water, and then lyophilized. The dried material was purified in a 30 g C18 column using a 5-50% acetonitrile (0.1% formic acid) gradient in water (0.1% formic acid) and lyophilized to obtain Compound 19-3 as a colorless solid (22.7 mg, 31% yield).

¹H NMR data of Compound 19-3 (2-PhPh-AQTGTGKT) was measured as follows:
¹H NMR (400 MHz; D2O): δ = 7.56-7.48 (m, 2H), 7.44-7.33 (m, 7H), 4.38-4.26 (m, 4H), 4.25-4.13 (m, 4H), 4.08 (d, J 3.9 Hz, 1H), 4.00-3.96 (m, 2H), 3.89 (s, 2H), 2.91 (t, J 7.5 Hz, 2H), 2.25 (t, J 7.5 Hz, 2H), 2.09-2.01 (m, 1H), 1.93-1.77 (m, 2H), 1.72-1.56 (m, 3H), 1.41-1.29 (m, 2H), 1.18 (d, J 7.2 Hz, 3H), 1.12 (d, J 5.6 Hz, 6H), 1.08 (d, J 6.4 Hz, 3H), 16 exchangeable protons not visible.

### 1.7.4. Synthesis of Ph-AQTGTGKT

For Ph-AQTGTGKT (Compound 19-4), Ph-AQTGTGKT, which is a final target compound, was synthesized by reacting Compound 17-4 obtained by the following Reaction Scheme 14 with Compound 14 according to Reaction Scheme 15.

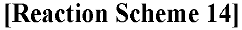

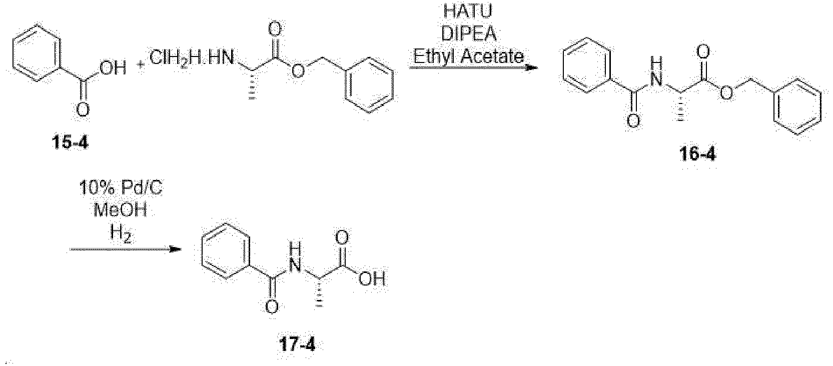

More specifically, H-Ala-OBzl.HCl (388 mg, 1.80 mmol, and 1.2 eq) was suspended in ethyl acetate (10 mL), and *N,N*-diisopropylethylamine (653 µL, 3.75 mmol, and 2.5 eq) was added thereto. After the resulting mixture was stirred at room temperature for 5 minutes, HATU (855 mg, 2.25 mmol, and 1.5 eq) and benzoic acid (183 mg, 1.50 mmol, and 1 eq) were added thereto, and the mixture was stirred at room temperature for 18 hours. The reaction mixture was diluted with ethyl acetate, and then washed with NH₄Cl (sat. aq.), NaHCO₃ (sat. aq.) and brine. The obtained organic material was dried (Na₂SO₄), filtered, and then concentrated under reduced pressure. The residue was purified by a 2-50% ethyl acetate gradient in heptane in a 25 g column to obtain Compound 16-4 as a colorless oil (375 mg, 88% yield).

Subsequently, 10% Pd/C (38 mg) soaked with a minimum amount of water was added to a solution of Ph-Ala-OBn (Compound 16-4; 375 mg, 1.32 mmol) in methanol (30 mL). The mixture was stirred under a hydrogen atmosphere (balloon) for 4 hours. Then, the mixture was filtered through a Celite pad and washed with methanol. The obtained filtrate was concentrated under reduced pressure to obtain Compound 17-4 as colorless glass (254 mg, 99% yield), which was reacted with Compound 14 according to the following Reaction Scheme 15.

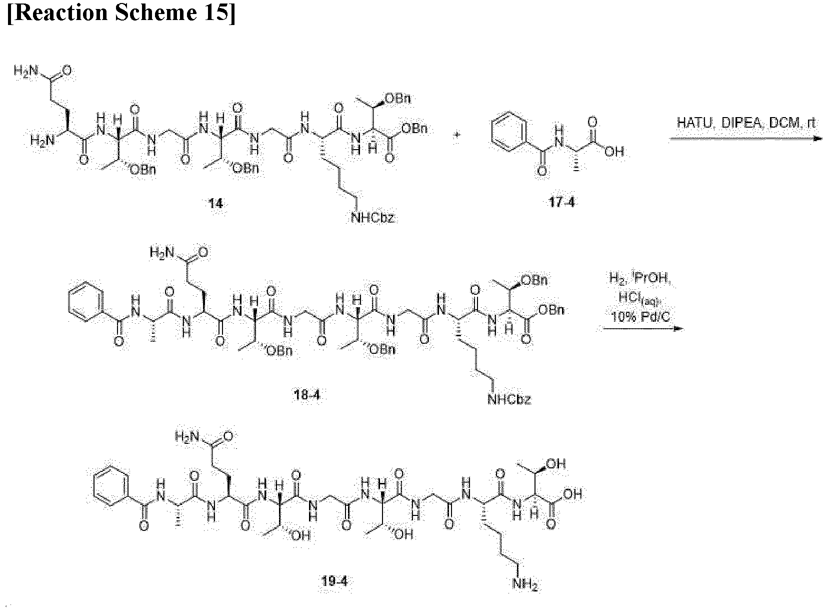

More specifically, HATU (106 mg, 0.278 mmol, and 1.1 eq) was added to a suspension of GlnThr(OBn)GlyThr(OBn)GlyLys(Cbz)Thr (OBn)OBn (Compound 14; 300 mg, 0.253 mmol, and 1 eq) and Ph-Ala-OH (Compound 17-4; 49.0 mg, 0.253 mmol, and 1 eq) in *N,N*-diisopropylethylamine (97.0 µL, 0.556 mmol, 2.2 eq) and dichloromethane (20 mL). The mixture was stirred at room temperature for 2 hours, and the reaction mixture was washed with NaHCO₃ (sat. aq.). The organic material was concentrated under reduced pressure, the residue was purified by a 40-100% acetonitrile (0.1% formic acid) gradient in water (0.1% formic acid) in a 60 g C18 column and lyophilized, and then Compound 18-4 as a colorless solid (200 mg, 58% yield) was obtained.

Subsequently, 10% Pd/C (110 mg) was added to a solution of Ph-AlaGlnThr(OBn)GlyThr(OBn)GlyLys(Cbz)Thr(OBn)OBn (Compound 18-4; 110 mg, 80.8 µmol) in 2 M hydrochloric acid (aqueous, 1.0 mL) and 2-propanol (20 mL). The mixture was stirred under a hydrogen atmosphere (balloon) for 18 hours. The mixture was filtered through a 0.45 µm syringe filter. The obtained filtrate was concentrated under reduced pressure, and the residue was dissolved in water, and then lyophilized. The dried material was purified in a 60 g C18 column using a 5-50% acetonitrile (0.1% formic acid) gradient in water (0.1% formic acid) and lyophilized to obtain Compound 19-4 as a colorless solid (23.2 mg, 33% yield).

¹H NMR data of Compound 19-4 (Ph-AQTGTGKT) was measured as follows:
¹H NMR (400 MHz; D2O): δ = 7.74-7.70 (m, 2H), 7.58-7.53 (m, 1H), 7.48-7.43 (m, 2H), 4.45-4.34 (m, 3H), 4.32-4.27 (m, 2H), 4.25-4.14 (m, 3H), 4.11 (d, J 3.8 Hz, 1H), 4.00-3.96 (m, 2H), 3.89 (s, 2H), 2.91 (t, J 7.4 Hz, 2H), 2.34 (t, J 7.6 Hz, 2H), 2.14-2.06 (m, 1H), 2.01-1.91 (m, 1H), 1.85-1.76 (m, 1H), 1.71-1.56 (m, 3H), 1.43 (d, J 7.3 Hz, 3H), 1.40-1.30 (m, 2H), 1.16-1.07 (m, 9H), 16 exchangeable protons not visible.

### 1.7.5. Synthesis of Naphthyl-AQTGTGKT

For Naphthyl-AQTGTGKT (Compound 19-5), Naphthyl-AQTGTGKT, which is a final target compound, was synthesized by reacting Compound 17-5 obtained by the following Reaction Scheme 16 with Compound 14 according to Reaction Scheme 17.

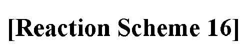

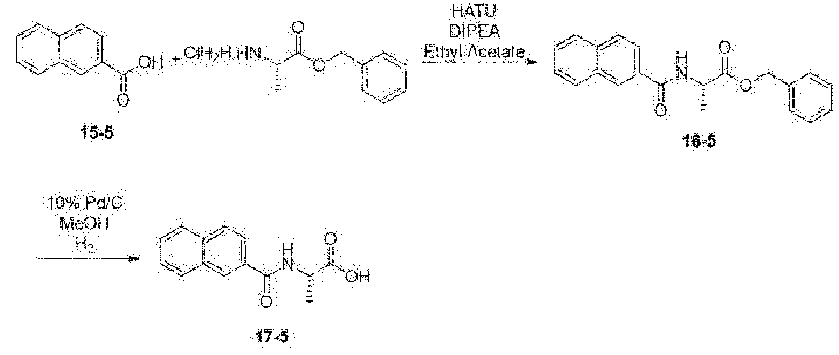

More specifically, H-Ala-OBzl.HCl (388 mg, 1.80 mmol, and 1.2 eq) was suspended in ethyl acetate (10 mL), and *N,N*-diisopropylethylamine (653 µL, 3.75 mmol, and 2.5 eq) was added thereto. After the resulting mixture was stirred at room temperature for 5 minutes, HATU (855 mg, 2.25 mmol, and 1.5 eq) and 2-naphthoic acid (258 mg, 1.50 mmol, and 1 eq) were added thereto, and the mixture was stirred at room temperature for 2 hours. The reaction mixture was diluted with ethyl acetate, and then washed with NH₄Cl (sat. aq.), NaHCO₃ (sat. aq.) and brine. The obtained organic material was dried (Na₂SO₄), filtered, and then concentrated under reduced pressure. The residue was purified by a 2-40% ethyl acetate gradient in heptane in a 25 g column to obtain Compound 16-5 as a colorless solid (385 mg, 77% yield).

Subsequently, 10% Pd/C (39 mg) soaked with a minimum amount of water was added to a solution of 2-Naphthyl-AlaOBn (Compound 16-5; 385 mg, 1.15 mmol) in methanol (20 mL). The mixture was stirred under a hydrogen atmosphere (balloon) for 4 hours. Then, the mixture was filtered through a Celite pad and washed with methanol. The obtained filtrate was concentrated under reduced pressure to obtain Compound 17-5 as a colorless solid (266 mg, 95% yield), which was reacted with Compound 14 according to the following Reaction Scheme 17.

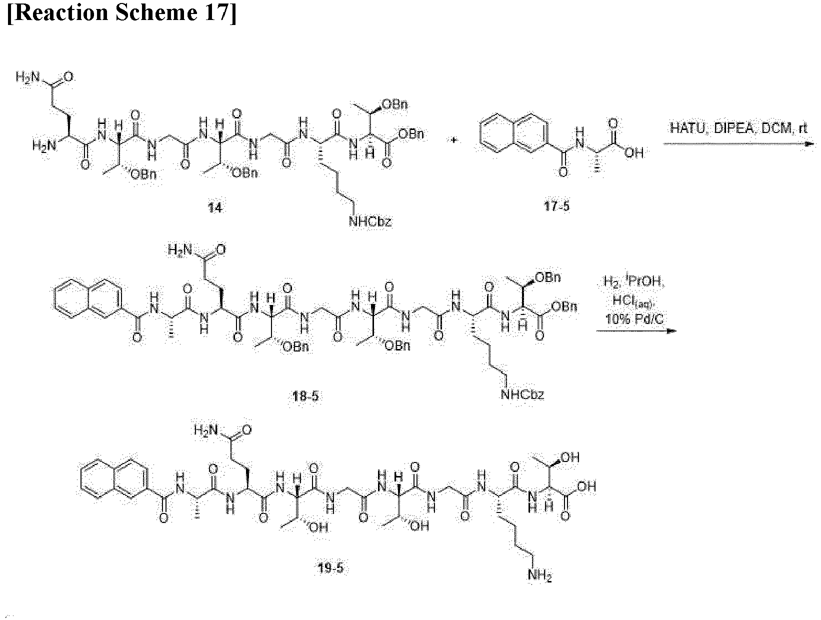

More specifically, HATU (106 mg, 0.278 mmol, and 1.1 eq) was added to a suspension of GlnThr(OBn)GlyThr(OBn)GlyLys(Cbz) Thr(OBn)OBn (Compound 14; 300 mg, 0.253 mmol, and 1 eq) and 2-Naphthyl-Ala-OH (Compound 17-5; 61.0 mg, 0.253 mmol, and 1 eq) in *N,N*-diisopropylethylamine (97.0 µL, 0.556 mmol, and 2.2 eq) and dichloromethane (20 mL). The mixture was stirred at room temperature for 2 hours, and subsequently washed with (sat. aq.) NaHCO₃. The obtained organic layer concentrated under reduced pressure, the residue was purified by a 40-100% acetonitrile (0.1% formic acid) gradient in water (0.1% formic acid) in a 60 g C18 column and lyophilized, and then Compound 18-5 as a colorless solid (230 mg, 64% yield) was obtained.

Subsequently, 10% Pd/C (101 mg) was added to a solution of 2-Naphthyl-AlaGlnThr(OBn)GlyThr(OBn)GlyLys(Cbz)Thr(OBn)OBn (Compound 18-5; 101 mg, 71.5 µmol) in 2 M hydrochloric acid (aqueous, 1.0 mL) and 2-propanol (20 mL). The mixture was stirred under a hydrogen atmosphere (balloon) for 3 hours. The mixture was filtered through a 0.45 µm syringe filter. The obtained filtrate was concentrated under reduced pressure, and the residue was dissolved in water, and then lyophilized. The dried material was purified on a 30 g C18 column using a 5-40% acetonitrile (0.1% formic acid) gradient in water (0.1% formic acid) and lyophilized to obtain Compound 19-5 as a colorless solid (23.2 mg, 33% yield).

¹H NMR data of Compound 19-5 (Naphthyl-AQTGTGKT) was measured as follows:
¹H NMR (400 MHz; D2O): δ = 8.32 (s, 1H), 8.01-7.90 (m, 3H), 7.79-7.73 (m, 1H), 7.64-7.55 (m, 2H), 4.51-3.70 (m, 13H), 2.96-2.81 (m, 2H), 2.39-2.30 (m, 2H), 2.18-2.04 (m, 1H), 2.03-1.93 (m, 1H), 1.85-1.72 (m, 1H), 1.71-1.53 (m, 3H), 1.50-1.43 (m, 3H), 1.39-1.24 (m, 2H), 1.19-1.04 (m, 9H), 16 exchangeable protons not visible.

### 1.8. Synthesis of Ac-AQTGTGKT

For Ac-AQTGTGKT (Compound 19-6), Ac-AQTGTGKT, which is a final target compound, was synthesized according to the following Reaction Scheme 18.

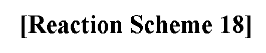

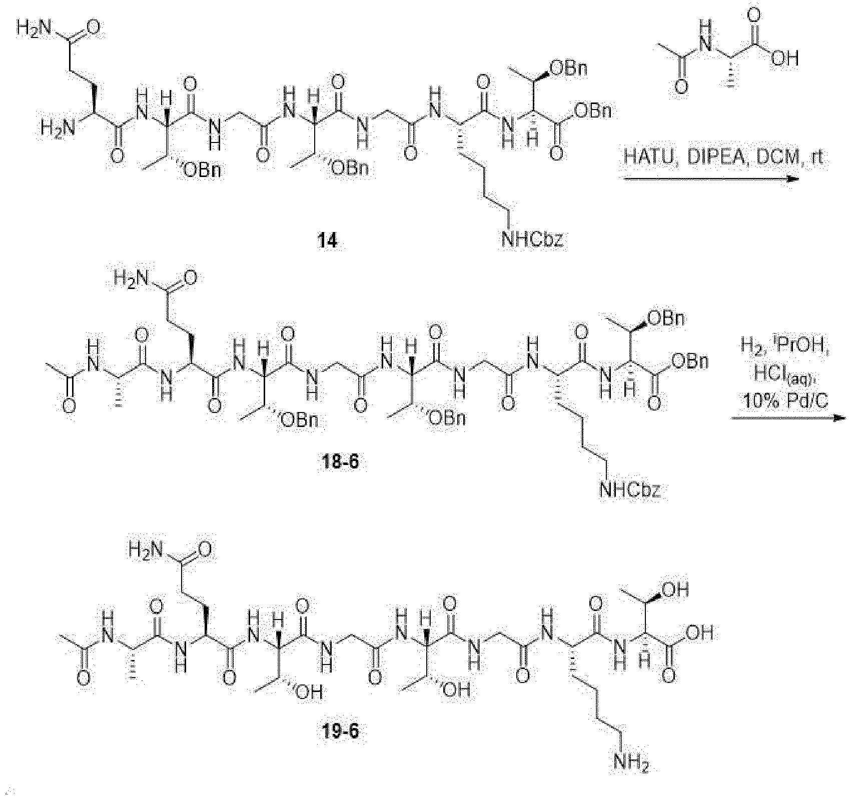

More specifically, HATU (112 mg, 0.294 mmol, and 1.2 eq) was added to a suspension of GlnThr(OBn)GlyThr(OBn)GlyLys(Cbz) Thr(OBn)OBn (Compound 14; 300 mg, 0.245 mmol, and 1 eq) and Ac-Ala-OH (32.1 mg, 0.245 mmol, and 1 eq) in *N, N*-diisopropylethylamine (94.0 µL, 0.540 mmol, and 2.2 eq) and dichloromethane (30 mL). The mixture was stirred at room temperature for 14 hours, and the reaction mixture was washed with (sat. aq.) NH₄Cl, NaHCO₃ and water. The organic layer was concentrated under reduced pressure, and the residue was purified by a 60 g C18 column in a 50-95% acetonitrile (0.1% formic acid) gradient in water (0.1% formic acid). The desired fractions were combined and lyophilized to obtain Compound 18-6 as a colorless solid (104 mg, 33% yield).

Subsequently, 10% Pd/C (10 mg) was added to a solution of Ac-AlaGlnThr(OBn)GlyThr(OBn)GlyLys(Cbz)Thr(OBn)OBn (Compound 18-6; 33 mg, 25 µmol) in 2 M hydrochloric acid (aqueous, 0.19 mL) and 2-propanol (5 mL). The mixture was stirred under a hydrogen atmosphere (balloon) for 14 hours. The mixture was filtered through a 0.45 µm syringe filter. The obtained filtrate was concentrated under reduced pressure, dissolved in water, and then lyophilized. The dried material was purified in a 500 mg SCX-2 cartridge while being eluted with 0.5 M ammonia in methanol. The desired fractions were combined, concentrated under reduced pressure, and then lyophilized to obtain Compound 19-6 as a colorless solid (7.6 mg, 37% yield).

¹H NMR data of Compound 19-6 (Ac-AQTGTGKT) was measured as follows:
¹H NMR (400 MHz; D2O): δ = 4.40-4.33 (m, 2H), 4.32-4.27 (m, 2H), 4.24-4.12 (m, 4H), 4.08 (d, J 4.0 Hz, 1H), 4.03-3.88 (m, 4H), 2.92 (t, J 7.2 Hz, 2H), 2.32 (t, J 7.8 Hz, 2H), 2.15-2.02 (m, 1H), 1.99-1.88 (m, 4H), 1.86-1.76 (m, 1H), 1.74-1.55 (m, 3H), 1.45-1.31 (m, 2H), 1.29 (t, J 7.4 Hz, 2H), 1.20-1.06 (m, 10H), 16 exchangeable protons not visible.

### 1.9. Synthesis of 4-PhPh-AQTGTGKT

### 1.9.1. Synthesis of QTGTGKT intermediate

An intermediate QTGTGKT for synthesizing 4-PhPh-AQTGTGKT was synthesized according to the following Reaction Schemes 19 to 23:

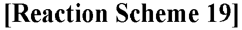

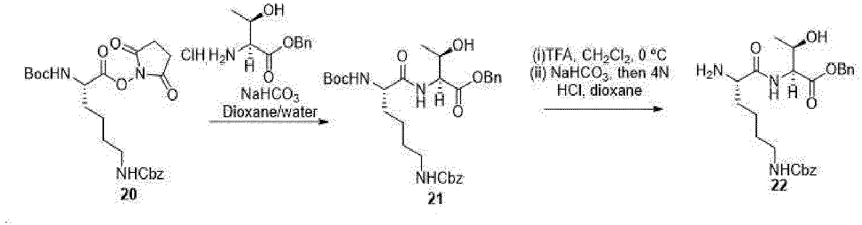

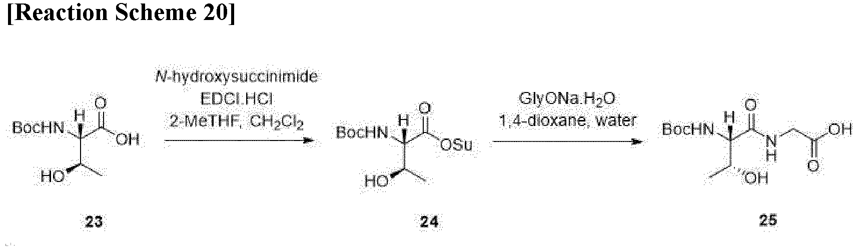

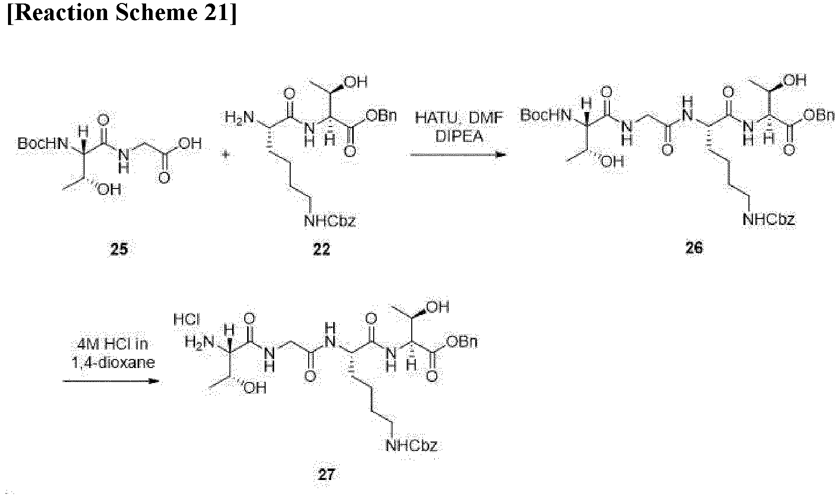

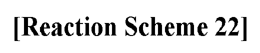

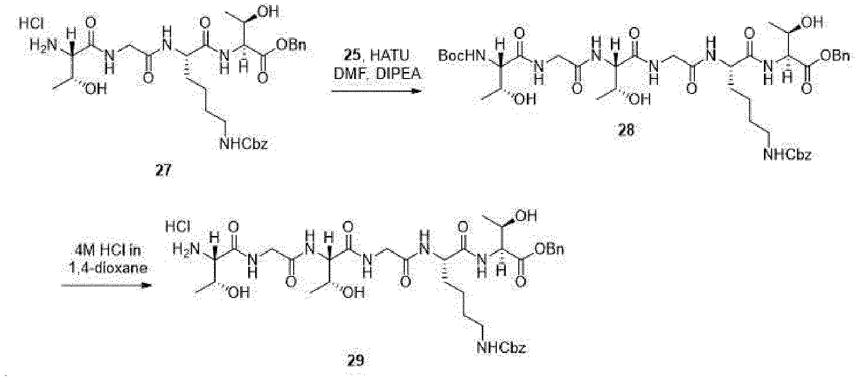

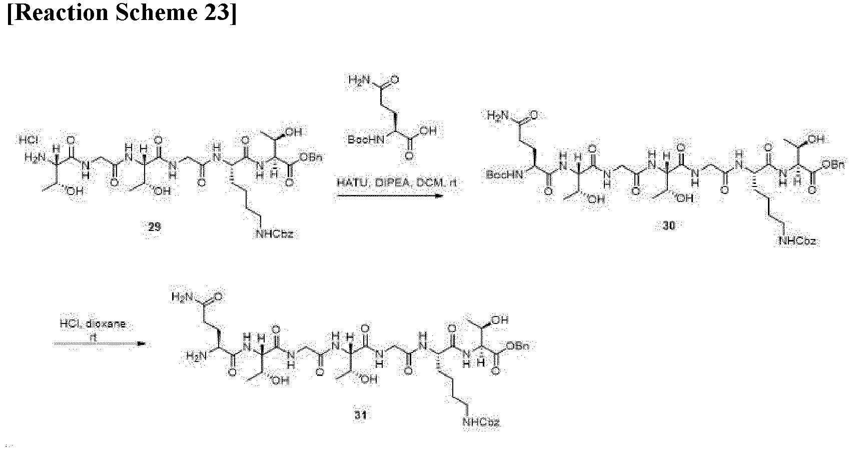

Reaction Schemes 19 to 23 are almost the same as Reaction Schemes 1 to 5 except for the presence or absence of a benzyl protecting group, and repeated descriptions thereof will be omitted.

### 1.9.2. Synthesis of 4-PhPh-AQTGTGKT

Compound 31 obtained in Reaction Scheme 23 was combined with an alanine derivative synthesized in the following Reaction Scheme 24 according to Reaction Scheme 25 to obtain a final product 4-PhPh-AQTGTGKT.

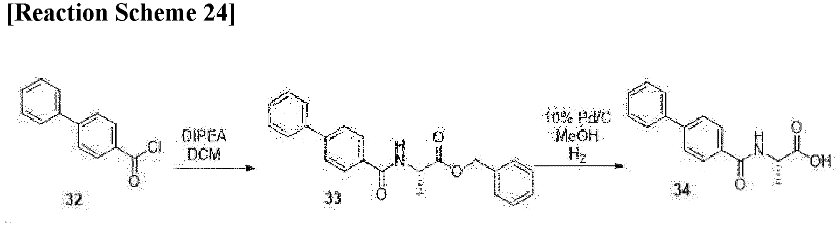

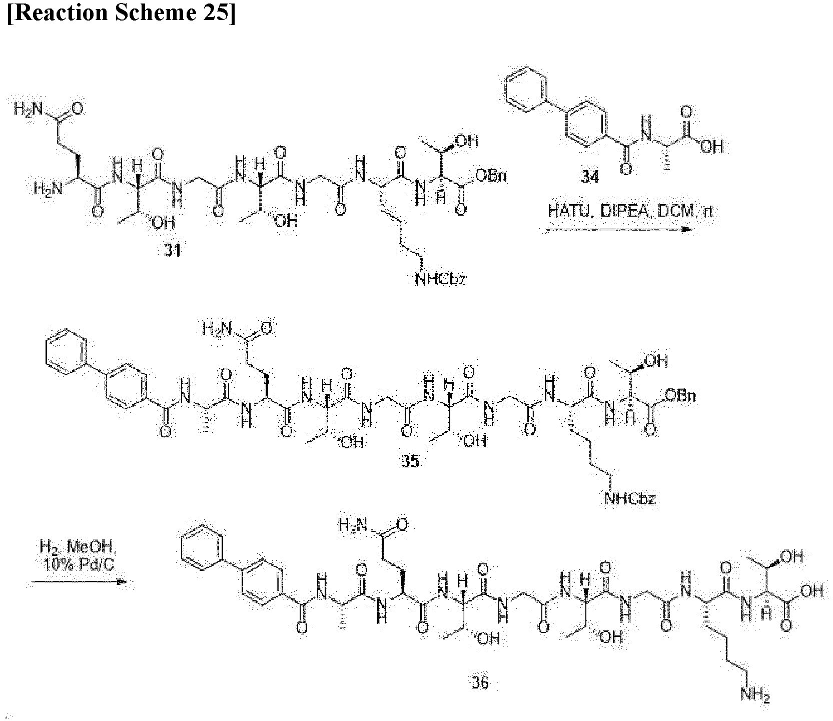

Since Reaction Schemes 24 and 25 were also carried out by a process similar to that of the above-described reaction schemes, repeated descriptions thereof will be omitted. Finally, Compound 36 as a colorless solid (583 mg, 68% yield) was obtained.

¹H NMR data of Compound 36 (4-PhPh-AQTGTGKT) was measured as follows:
¹H NMR (400 MHz; D2O): δ = 7.85 (d, J 8.8 Hz, 2H), 7.77 (d, J 8.8 Hz, 2H), 7.70 (d, J 7.2 Hz, 2H), 7.49 (t, J 7.2 Hz, 2H), 7.42 (t, J 7.1 Hz, 1H), 4.34-4.04 (m, 6H), 4.07 (d, J 3.6 Hz, 1H), 3.93 (d, J 2.0 Hz, 2H), 2.82 (t, J 7.0 Hz, 2H), 1.82-1.67 (m, 1H), 1.66-1.55 (m, 1H), 1.54-1.44 (m, 2H), 1.37-1.22 (m, 2H), 1.18 (d, J 6.4 Hz, 3H), 1.06 (t, J 6.5 Hz, 3H), 10 exchangeable protons not visible.

### 1.10. Confirmation of compound

Chemical structures thereof were confirmed by analyzing seven AQTGTGKT analogs obtained by the above preparation method by an ¹H NMR and ultraperformance liquid chromatography-mass spectrometry (UPLC-MS) technique. The analysis results are shown in FIGS. 1 to 7, and the compounds are summarized in Table 1.

**[Table 1]**

| **Title** | **Chemical Formula** |
|---|---|
| AQTGTGKT | 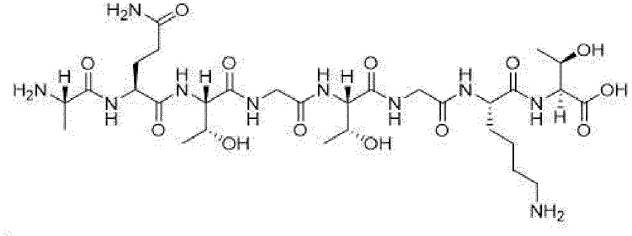 |
| 4-PhPh-AQTGTGKT | 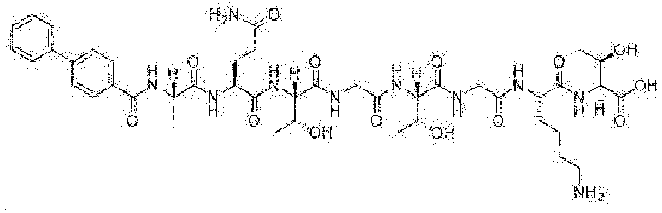 |
| Ac-AQTGTGKT | 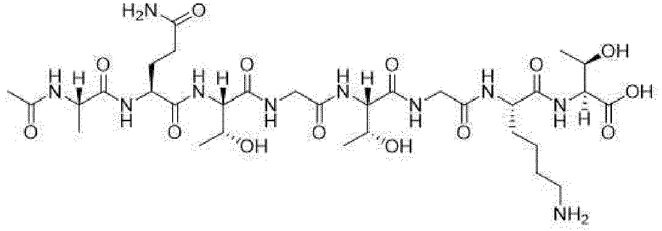 |
| 3-PhPh-AQTGTGKT | 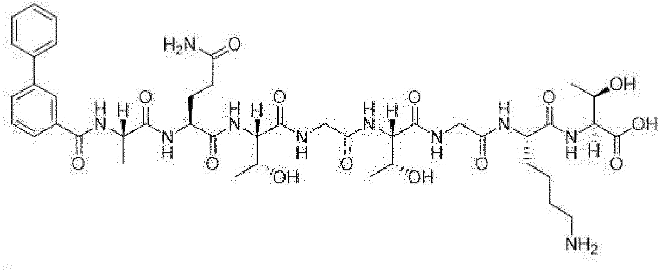 |
| 4-MeOPh-AQTGTGKT | 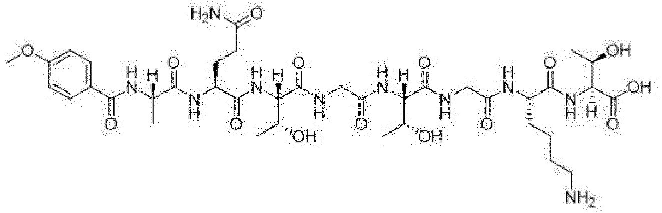 |
| 2-PhPh-AQTGTGKT | 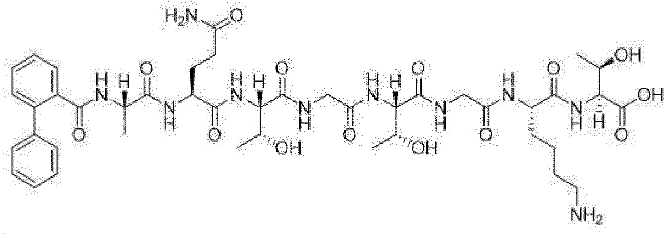 |
| Ph-AQTGTGKT | 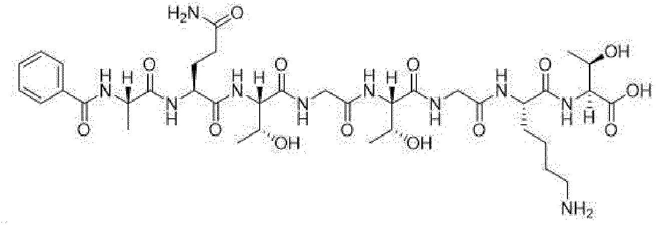 |
| Naphthyl-AQTGTGKT | 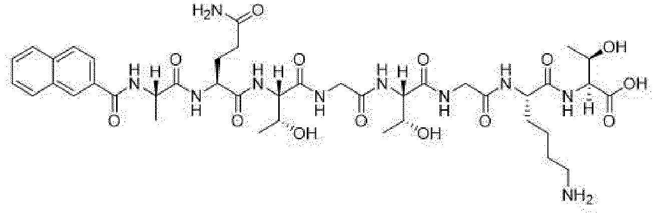 |

### Example 2: Confirmation of anticancer effects of AQTGTGKT analog

### 2.1. Suppression effects on lung cancer cell viability

After a lung cancer cell line H1299 was treated with the AQTGTGKT analogs at a concentration of 100 µM for 48 hours or a lung cancer cell line H820 or H1975 was treated with the AQTGTGKT analogs at a concentration of 10 µM for 72 hours according to the CTG assay method described in the experimental method, the cytotoxicities of respective analogs were compared.

As a result, as illustrated in FIG. 8, when the cell line was treated with 4-PhPh-AQTGTGKT, the activity of cells was reduced by about 10% compared to the control (AQTGTGKT), and Ac-AQTGTGKT showed a cell viability suppression effect of 4.2%, 3-PhPh-AQTGTGKT showed a cell viability suppression effect of 15%, 4-MeOPh-AQTGTGKT showed a cell viability suppression effect of 12%, 2-PhPh-AQTGTGKT showed a cell viability suppression effect of 20%, Ph-AQTGTGKT showed a cell viability suppression effect of 18%, and Naphthyl-AQTGTGKT showed a cell viability suppression effect of 14%.

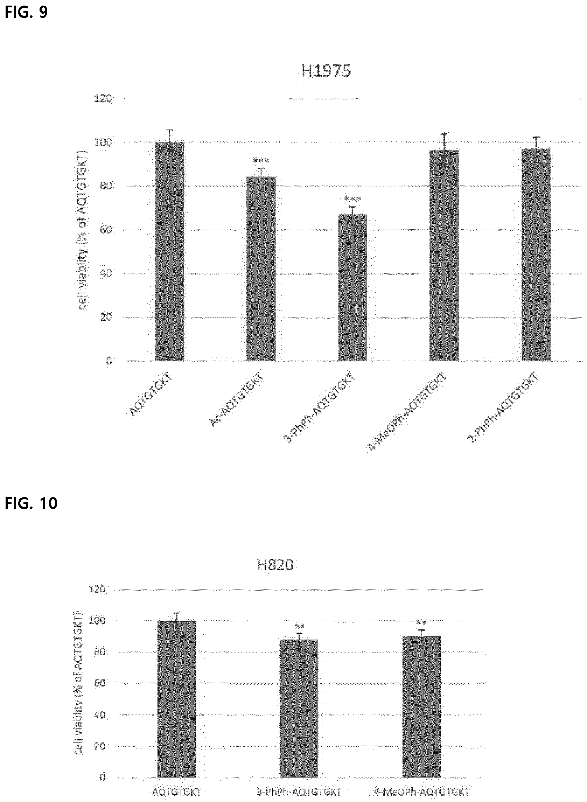

In the case of the H1975 cell line, as illustrated in FIG. 9, when the cell line was treated with Ac-AQTGTGKT, the activity of cells was reduced by about 16% compared to the control (AQTGTGKT), and 3-PhPh-AQTGTGKT showed a cell viability suppression effect of 33%, 4-MeOPh-AQTGTGKT showed a cell viability suppression effect of 4%, and 2-PhPh-AQTGTGKT showed a cell viability suppression effect of 3%.

Further, in the case of the H820 cell line, as illustrated in FIG. 10, when the cell line was treated with 3-PhPh-AQTGTGKT, the activity of cells was reduced by about 12% compared to the control (AQTGTGKT), and 4-MeOPh-AQTGTGKT showed a cell viability suppression effect of 10%.

These results exhibit that the amidation analogs can suppress the division of lung cancer cells very effectively.

### 2.2. Suppression effect on activity and proliferation of breast cancer cells

### (1) Suppression effects on activity of breast cancer cells

After a breast cancer cell line MDA-MB-231 was treated with the AQTGTGKT analogs at a concentration of 100 µM for 48 hours according to the CTG assay method described in the experimental method, the activity of cells was compared by measuring the amount of ATP in cells.

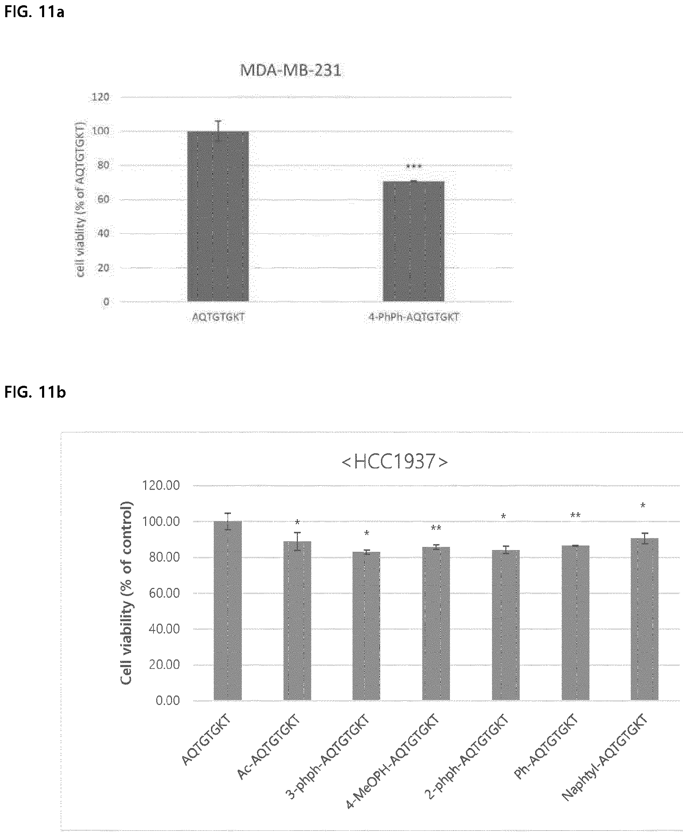

As a result, as illustrated in FIG. 11a, when the cell line was treated with 4-PhPh-AQTGTGKT, the activity of cells showed a reduction of about 30% compared to the control (AQTGTGKT), proving that the treatment has an excellent suppression effect on the division of breast cancer cells.

### (2) Suppression effects on growth and proliferation ability of breast cancer cells

Cell proliferation suppression effects were compared by treating a breast cancer cell line with the AQTGTGKT analogs according to the MTT assay method described in the experimental method. HCC1937 was used as the breast cancer cell line.

The HCC1937 cell line was treated with Ac-AQTGTGKT, 3-PhPh-AQTGTGKT, 4-MeOPh-AQTGTGKT, 2-PhPh-AQTGTGKT, Ph-AQTGTGKT, and Naphthyl-AQTGTGKT at a concentration of 100 µM, respectively, and cell proliferation suppression effects were compared after 72 hours. As a result, as illustrated in FIG. 11B, it was shown that Ac-AQTGTGKT, 3-PhPh-AQTGTGKT, 4-MeOPh-AQTGTGKT, 2-PhPh-AQTGTGKT, Ph-AQTGTGKT, and Naphthyl-AQTGTGKT suppressed the proliferation of cells by about 11.1%, 17.1%, 14.3%, 15.9%, 13.6%, and 9.5%, respectively, compared to the control (AQTGTGKT).

The experimental results described above indicate that the AQTGTGKT analogs according to the present invention exert an excellent anticancer effect on breast cancer.

### 2.3. Suppression effects on growth and proliferation ability of blood cancer cells

Cytotoxicity was compared by treating a blood cancer cell line with the AQTGTGKT analogs according to the MTT assay method described in the experimental method. As the blood cancer cell line, Jurkat clone E6-1 was used.

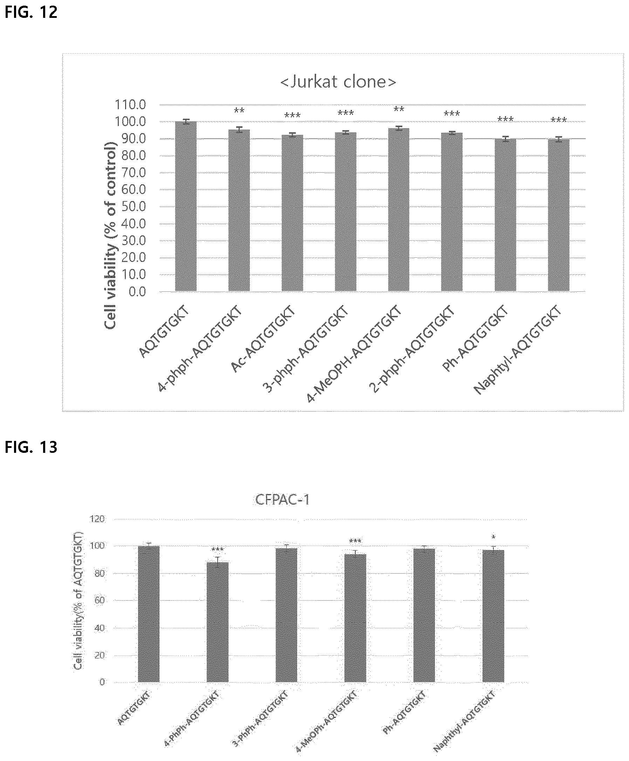

Jurkat clone E6-1 was treated with 4-PhPh-AQTGTGKT, Ac-AQTGTGKT, 3-PhPh-AQTGTGKT, 4-MeOPh-AQTGTGKT, 2-PhPh-AQTGTGKT, Ph-AQTGTGKT, and Naphthyl-AQTGTGKT at a concentration of 10 µM, respectively, and cell proliferation suppression effects were compared after 48 hours. As a result, as illustrated in FIG. 12, it was shown that 4-PhPh-AQTGTGKT, Ac-AQTGTGKT, 3-PhPh-AQTGTGKT, 4-MeOPh-AQTGTGKT, 2-PhPh-AQTGTGKT, Ph-AQTGTGKT, and Naphthyl-AQTGTGKT suppressed the proliferation of cells by about 4.8%, 7.9%, 6.4%, 4%, 6.7%, 10.2%, and 10.4%, respectively, compared to the control (AQTGTGKT).

The results described above indicate that the AQTGTGKT analogs according to the present invention have an excellent anticancer effect on blood cancer cells.

### 2.4. Suppression effects on growth and proliferation ability of pancreatic cancer cells

Cytotoxicity was compared by treating a pancreatic cancer cell line CFPAC-1 with the AQTGTGKT analogs at a concentration of 10 µM for 72 hours according to the MTT assay method described in the experimental method.

As a result, as illustrated in FIG. 13, when the cell line was treated with 4-PhPh-AQTGTGKT, the activity of cells was reduced by about 12% compared to the control (AQTGTGKT), and 3-PhPh-AQTGTGKT showed a cell viability suppression effect of 2%, 4-MeOPh-AQTGTGKT showed a cell viability suppression effect of 6%, Ph-AQTGTGKT showed a cell viability suppression effect of 3%, and Naphthyl-AQTGTGKT showed a cell viability suppression effect of 3%. The results described above indicate that the AQTGTGKT analogs according to the present invention have an excellent anticancer effect on pancreatic cancer cells.

### 2.5. Suppression effects on growth and proliferation ability of colorectal cancer cells

Cytotoxicity was compared by treating a colorectal cancer cell line HT29 with the AQTGTGKT analogs at a concentration of 50 µM for 72 hours according to the MTT assay method described in the experimental method.

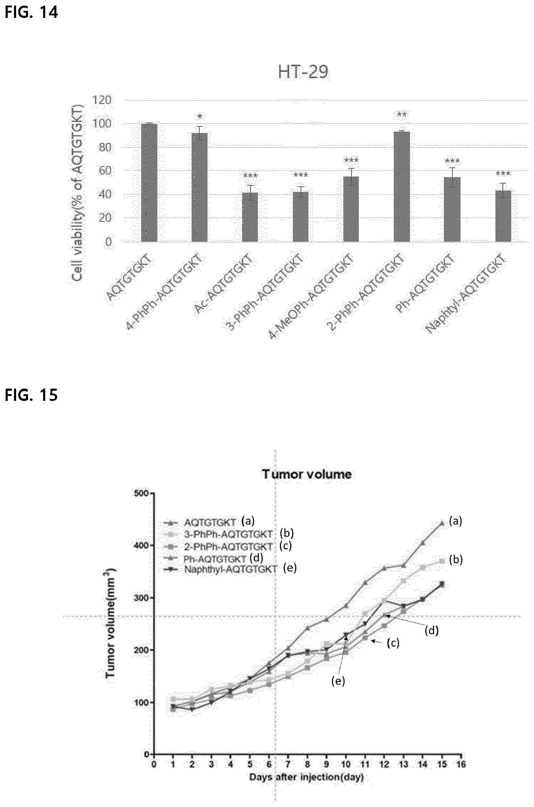

As a result, as illustrated in FIG. 14, when the cell line was treated with 4-PhPh-AQTGTGKT, the proliferation of cells was reduced by about 8% compared to the control (AQTGTGKT), and Ac-AQTGTGKT showed a cell proliferation suppression effect of 58%, 3-PhPh-AQTGTGKT showed a cell proliferation suppression effect of 59%, 4-MeOPh-AQTGTGKT showed a cell proliferation suppression effect of 48%, 2-PhPh-AQTGTGKT showed a cell proliferation suppression effect of 7%, Ph-AQTGTGKT showed a cell proliferation suppression effect of 40%, and Naphthyl-AQTGTGKT showed a cell proliferation suppression effect of 58%. The results described above indicate that the AQTGTGKT analogs according to the present invention have an excellent anticancer effect on colorectal cancer cells.

### 2.6. Tumor growth suppression effect xenograft animal model with lung cancer cell lines

Tumor growth was compared by treating nude mice inoculated with a lung cancer cell line H820 with the AQTGTGKT analogs at a dose of 10 mpk 5 times at 3-day intervals and 5 times at 2-day intervals for a total of 10 times according to the animal experimental analysis method described in the experimental method.

As a result, as illustrated in FIG. 15, when the mice with the inoculated H820 lung cancer cell line were treated with 3-PhPh-AQTGTGKT, tumor growth was reduced by about 16.5% compared to the control (AQTGTGKT), and 2-PhPh-AQTGTGKT showed a tumor growth suppression effect of 27%, Ph-AQTGTGKT showed a tumor growth suppression effect of 20%, and Naphthyl-AQTGTGKT showed a tumor growth suppression effect of 26%.

In addition, tumor growth was compared by treating nude mice inoculated with H1975 lung cancer cells with the AQTGTGKT analogs at a dose of 10 mpk for 14 days.

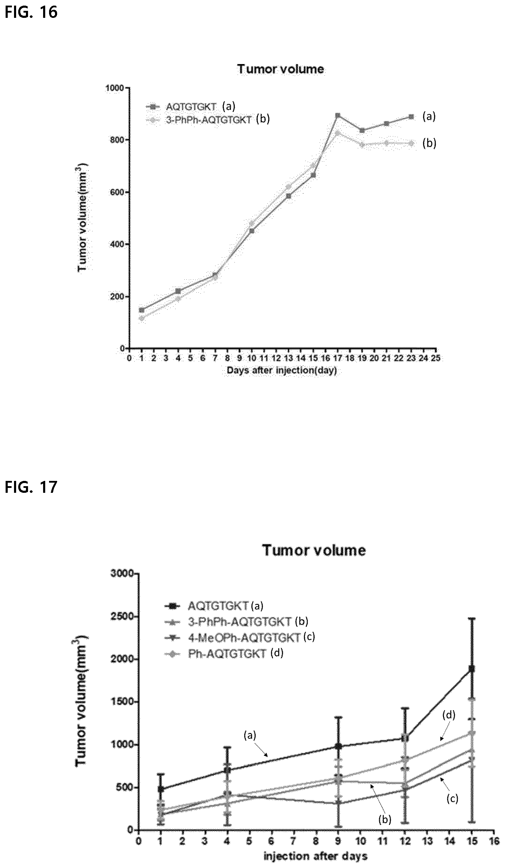

As a result, as illustrated in FIG. 16, when the nude mice were treated with 3-PhPh-AQTGTGKT, the growth of tumors was reduced by about 16.5% compared to the control (AQTGTGKT), thereby showing an suppression effect on tumor growth.

Collectively, the results as described above show that in the animal model inoculated with lung cancer cells, the compound of the present invention has an excellent anticancer effect.

### 2.7. Tumor growth suppression effect in xenograft animal model with lung cancer cell lines

Tumor growth was compared by administering AQTGTGKT and three types of AQTGTGKT analogs (3-PhPh-AQTGTGKT, 4-MeOPh-AQTGTGKT, and Ph-AQTGTGKT) to the tail veins of immunodeficient mice inoculated with a breast cancer cell line at a dose of 10 mpk at 2-day intervals for a total of 7 times according to the animal experimental analysis method described in the experimental method.

As a result of calculating the tumor volume on day 15 which is 2 days after completing the administration of analogs 7 times, the AQTGTGKT administration group was measured to be 1884.17 mm³ on average, and the 3-PhPh-AQTGTGKT administration group, the 4-MeOPh-AQTGTGKT administration group, and the Ph-AQTGTGKT administration group were measured to be 944.70 mm³, 812.69 mm³, and 1133.80 mm³, respectively, and suppressed the tumor growth by about 49.86%, 56.86%, and 39.82%, respectively, compared to the tumor volume of the AQTGTGKT group (FIG. 17).

Collectively, the results as described above show that in the animal model inoculated with lung cancer cells, the compounds of the present invention have an excellent anticancer effect without affecting the body weights of the mice.

### 2.8. Tumor growth suppression effect in xenograft animal model with colorectal cancer cells

Tumor growth was compared by administering AQTGTGKT and two types of AQTGTGKT analogs (3-PhPh-AQTGTGKT and Naphthyl-AQTGTGKT) to the tail veins of mice inoculated with a CT26 cell line that had induced the expression of a CAGE gene at a dose of 10 mpk at 2-day intervals for a total of 7 times according to the animal experimental analysis method described in the experimental method.

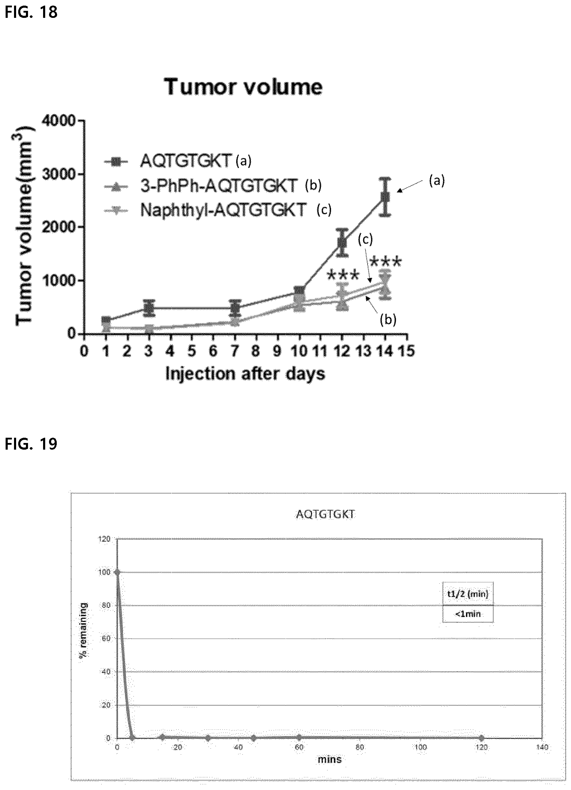

As a result, it was shown that the tumor growth of the analog administration groups was suppressed after day 10 compared to the control (AQTGTGKT administration group), and as a result of analyzing the tumor volume on day 14 which is the day after the 7^{th} administration of the analogs, it was confirmed that the AQTGTGKT administration group was measured to bet 2573.07 mm³ on average, the 3-PhPh-AQTGTGKT administration group was measured to be 880.35 mm³ on average, and the Naphthyl-AQTGTGKT administration group was measured to be 880.35 mm³ (FIG. 18A). It was shown that the tumor growth of the 3-PhPh-AQTGTGKT administration group and the Naphthyl-AQTGTGKT administration group was suppressed by about 65.79% and about 62.04%, respectively, compared to the AQTGTGKT administration group. Based on days 12 and 14, the tumor growth in the groups injected two types of analogs 3-PhPh-AQTGTGKT and Naphthyl-AQTGTGKT was statistically significantly suppressed with p<0.001 compared to the AQTGTGKT administration group.

Collectively, the results as described above shown that in the animal model inoculated with CT26 cells that induce the expression of CAGE, the compounds of the present invention have an excellent anticancer effect without affecting the body weights of the mice.

### Example 3: Confirmation of stability of AQTGTGKT analog in blood

In order to confirm how long minutes the AQTGTGKT analogs according to the present invention were stably present in blood, 10 µM of each compound was put into 100% human blood, and the residual amount in blood was measured at time points when 0, 5, 10, 15, 30, 60 and 120 minutes had elapsed.

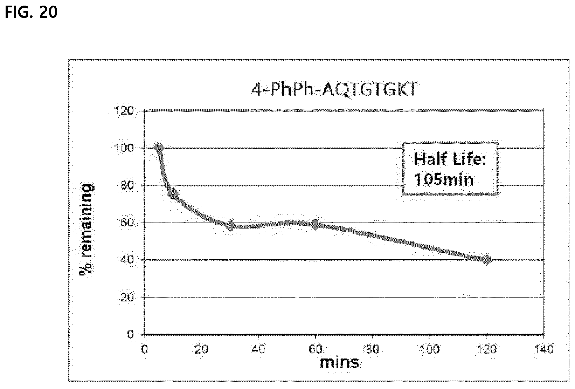

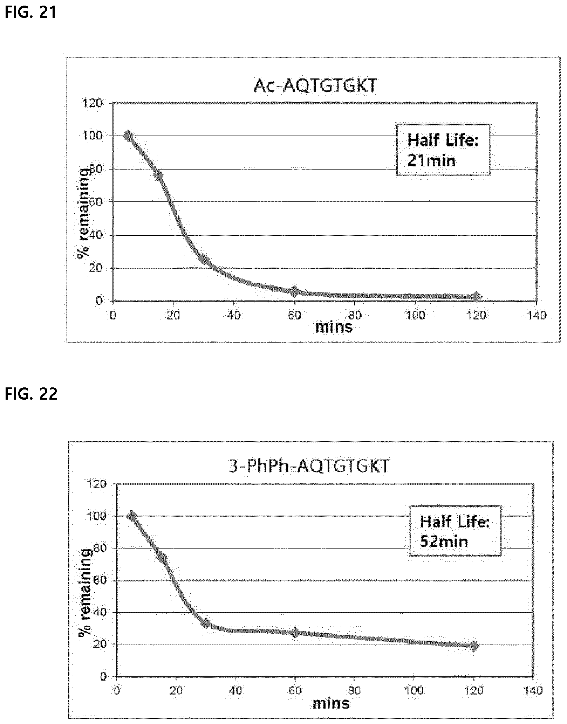

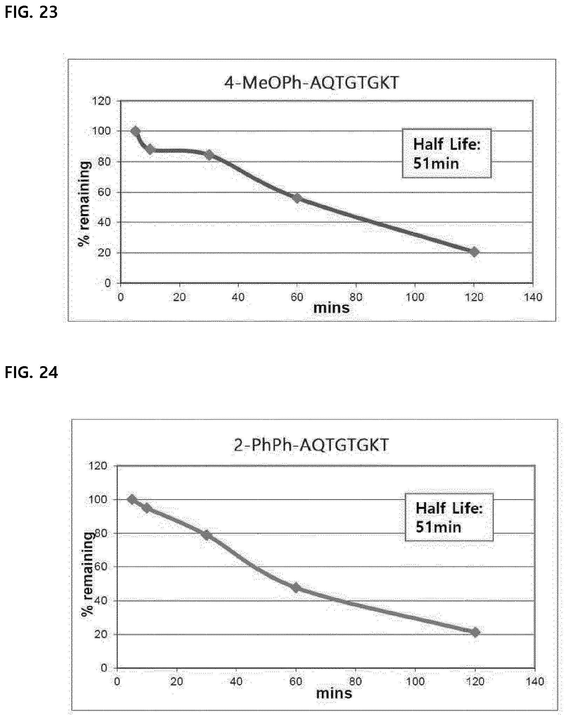

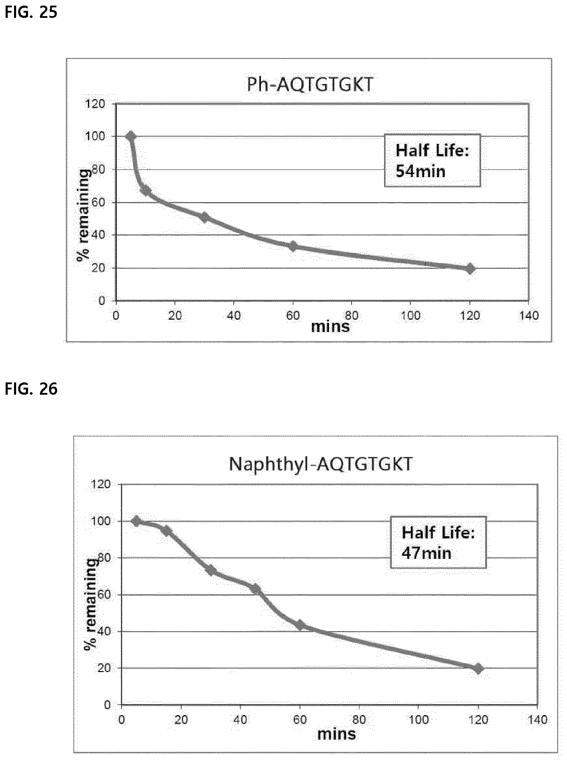

As a result, as illustrated in FIGS. 19 to 26, it was confirmed that the half-life of AQTGTGKT was less than 1 minute and almost no residual AQTGTGKT was detected because almost all of the AQTGTGKT was degraded in the blood within 20 minutes. In contrast, all 7 types of AQTGTGKT analogs according to the present invention showed remarkably improved stability, and in particular, 4-PhPh-AQTGTGKT, 3-PhPh-AQTGTGKT, 4-MeOPh-AQTGTGKT, 2-PhPh-AQTGTGKT, Ph-AQTGTGKT and Naphthyl-AQTGTGKT showed a half-life of 47 minutes to 105 minutes, and 20 to 40% of the analogs remained in blood even after 120 minutes had elapsed, thereby showing high stability. These results showed that amidation analogs exhibited not only a superior anticancer effect, but also much improved stability in blood, and thus may be stably present without being degraded until reaching a target.

The present invention relates to an analog compound of a novel oligopeptide AQTGTGKT, a pharmaceutical composition for preventing or treating cancer, including the same as an active ingredient and a preparation method thereof, and it was confirmed that the analog of the oligopeptide AQTGTGKT exhibited an excellent anticancer effect, and was stably present in human blood. Therefore, since the pharmaceutical composition according to the present invention exhibits an excellent effect of suppressing the proliferation of cancer cells in addition to the fact that there is less concern about immune responses and the pharmaceutical composition easily penetrates into tissue due to smaller molecular weights of oligopeptides than those of antibodies, which is an advantage of the oligopeptides, and an effect of being able to be stably present in human blood, the pharmaceutical composition is expected to be used as a useful anticancer agent for treating cancer.

The above description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only exemplary in all aspects and are not restrictive. The invention is set out in the appended set of claims.

## Claims

1. A compound represented by the following General Formula:
[General Formula] X-AQTGTGKT
(in General Formula,
A is alanine, Q is glutamine, T is threonine, G is glycine, and K is lysine, and
the X is one or more selected from the group consisting of

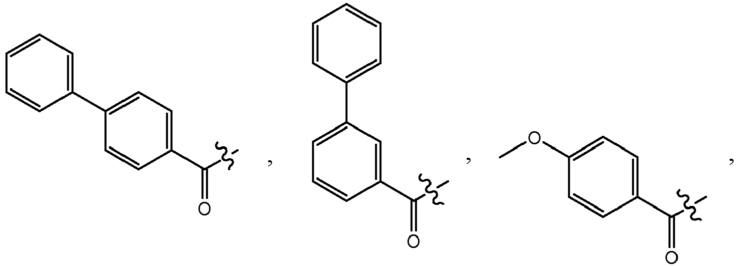

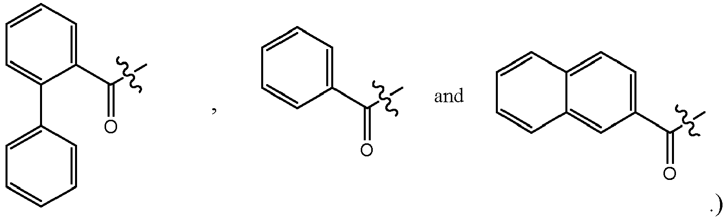

2. A compound for use in the prevention or treatment of cancer, wherein the compound is represented by the following General Formula:
[General Formula] X-AQTGTGKT
(in General Formula,
A is alanine, Q is glutamine, T is threonine, G is glycine, and K is lysine, and
the X is one or more selected from the group consisting of

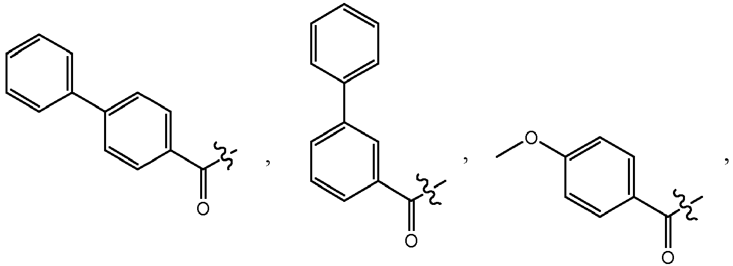

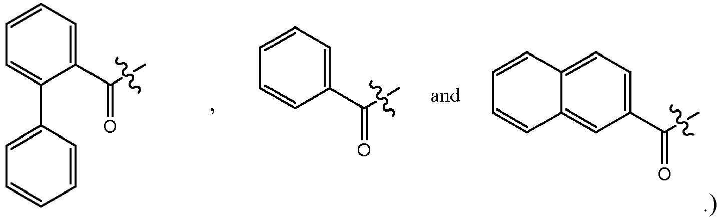

3. The compound for use of claim 2, wherein the cancer is a cancer selected from the group consisting of lung cancer, breast cancer, blood cancer, colorectal cancer, pancreatic cancer and combinations thereof.

4. The compound for use of claim 2 or 3, wherein the cancer in lung cancer.

5. The compound for use of claim 3 or 4, wherein the lung cancer is non-small cell lung cancer.

6. The compound for use of claim 2 or 3, wherein the cancer is blood cancer.

7. The compound for use of claim 3 or 6, wherein the blood cancer is a blood cancer selected from the group consisting of leukemia, lymphoma, multiple myeloma, and combinations thereof.

8. The compound for use of claim 2 or 3, wherein the cancer is breast cancer.

9. The compound for use of claim 2 or 3, wherein the X is one or more selected from the group consisting of 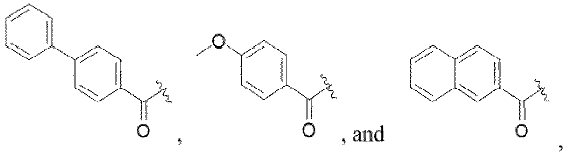 and the cancer is pancreatic cancer.

10. The compound for use of claim 2 or 3, wherein the cancer is colorectal cancer.

11. The compound for use of any one of claims 2 to 10, wherein when X is 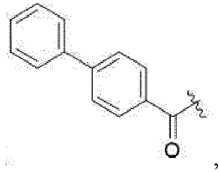 and a half-life of the compound in human blood is 100 minutes to 150 minutes.

12. The compound for use of any one of claims 2 to 10, wherein when X is one or more selected from the group consisting of 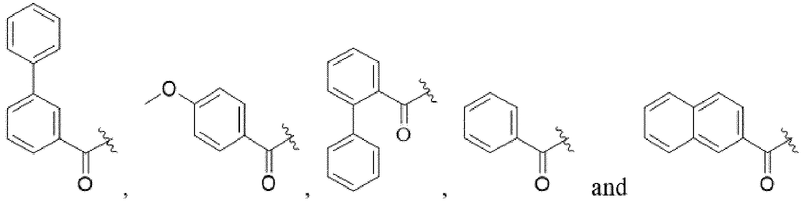 , and a half-life of the compound in human blood is 45 minutes to 70 minutes.

13. A method for preparing an oligopeptide X-AQTGTGKT, the method comprising the following steps:
(A is alanine, Q is glutamine, T is threonine, G is glycine, and K is lysine, and
X is one selected from the group consisting of 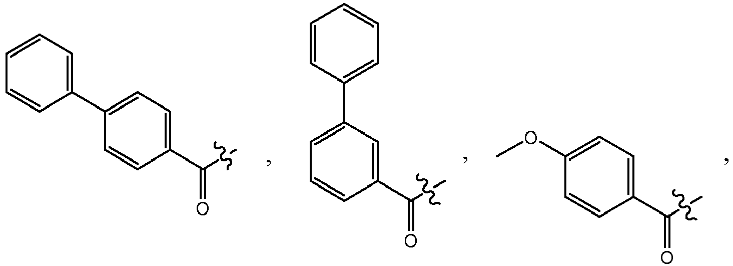 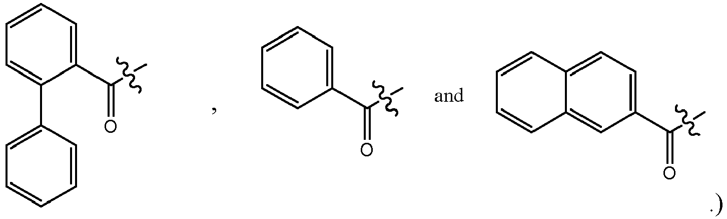
(1) synthesizing each of TG and KT;
(2) synthesizing TGKT by combining TG and KT;
(3) synthesizing TGTGKT by bonding TG to the N-terminal of the TGKT;
(4) synthesizing QTGTGKT by bonding Q to the N-terminal of the TGTGKT; and
(5) synthesizing X-AQTGTGKT by bonding an alanine derivative (X-A) to the N-terminal of the QTGTGKT.

## Patentansprüche

1. Verbindung, dargestellt durch die folgende Allgemeine Formel:
[Allgemeine Formel] X-AQTGTGKT
(in der Allgemeinen Formel
ist A Alanin, Q ist Glutamin, T ist Threonin, G ist Glycin und K ist Lysin, und X ist eines oder mehrere, ausgewählt aus der Gruppe bestehend aus

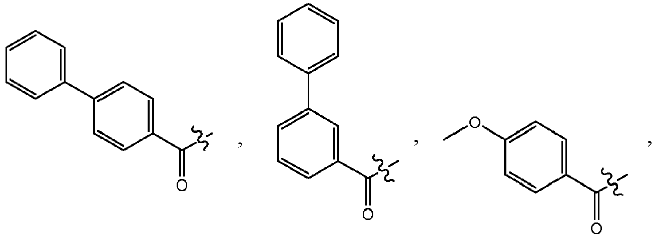

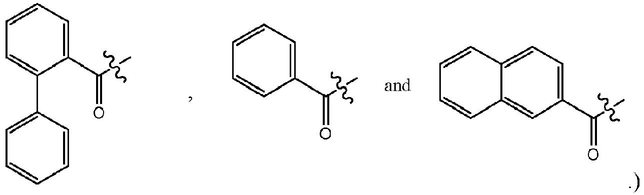

2. Verbindung zur Verwendung bei der Prävention oder Behandlung von Krebs, wobei die Verbindung durch die folgende Allgemeine Formel dargestellt wird:
[Allgemeine Formel] X-AQTGTGKT
(in der Allgemeinen Formel
ist A Alanin, Q ist Glutamin, T ist Threonin, G ist Glycin und K ist Lysin, und X ist eines oder mehrere, ausgewählt aus der Gruppe bestehend aus

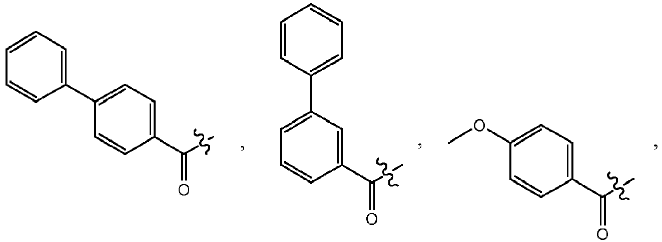

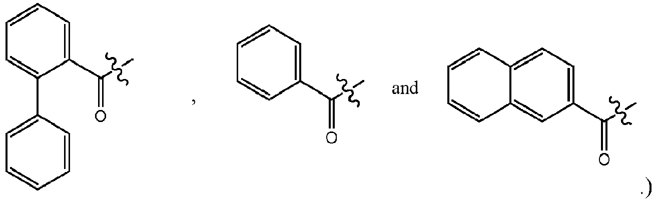

3. Verbindung zur Verwendung nach Anspruch 2, wobei der Krebs ein Krebs ist, der aus der Gruppe bestehend aus Lungenkrebs, Brustkrebs, Blutkrebs, kolorektalem Krebs, Bauchspeicheldrüsenkrebs und Kombinationen davon ausgewählt ist.

4. Verbindung zur Verwendung nach Anspruch 2 oder 3, wobei der Krebs Lungenkrebs ist.

5. Verbindung zur Verwendung nach Anspruch 3 oder 4, wobei der Lungenkrebs ein nicht-kleinzelliger Lungenkrebs ist.

6. Verbindung zur Verwendung nach Anspruch 2 oder 3, wobei der Krebs Blutkrebs ist.

7. Verbindung zur Verwendung nach Anspruch 3 oder 6, wobei der Blutkrebs ein Blutkrebs ist, der aus der Gruppe bestehend aus Leukämie, Lymphom, multiplem Myelom und Kombinationen davon ausgewählt ist.

8. Verbindung zur Verwendung nach Anspruch 2 oder 3, wobei der Krebs Brustkrebs ist.

9. Verbindung zur Verwendung nach Anspruch 2 oder 3, wobei X eines oder mehrere ist, ausgewählt aus der Gruppe bestehend aus 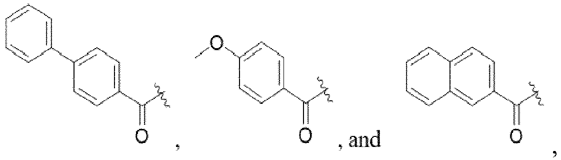 und der Krebs Bauchspeicheldrüsenkrebs ist.

10. Verbindung zur Verwendung nach Anspruch 2 oder 3, wobei der Krebs kolorektaler Krebs ist.

11. Verbindung zur Verwendung nach einem der Ansprüche 2 bis 10, wobei, wenn X 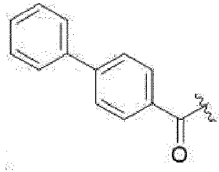 ist, und eine Halbwertszeit der Verbindung im menschlichen Blut 100 Minuten bis 150 Minuten beträgt.

12. Verbindung zur Verwendung nach einem der Ansprüche 2 bis 10, wobei, wenn X eines oder mehrere, ausgewählt aus der Gruppe bestehend aus 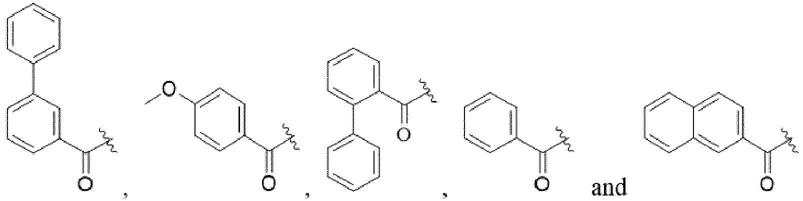 ist, und eine Halbwertszeit der Verbindung im menschlichen Blut 45 Minuten bis 70 Minuten beträgt.

13. Verfahren zur Herstellung eines Oligopeptids X-AQTGTGKT, wobei das Verfahren die folgenden Schritte umfasst:
(A ist Alanin, Q ist Glutamin, T ist Threonin, G ist Glycin und K ist Lysin, und
X ist eines ausgewählt aus der Gruppe bestehend aus 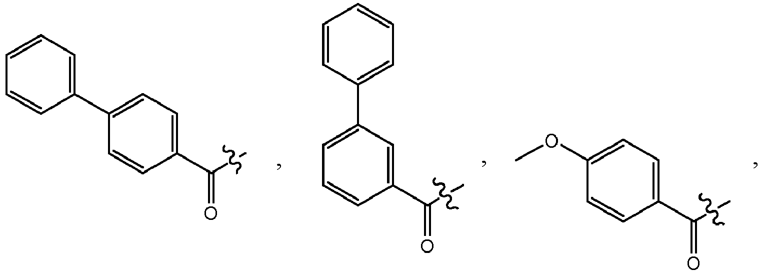 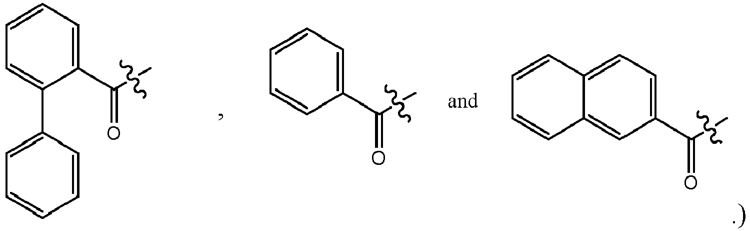
(1) Synthetisieren von TG und KT;
(2) Synthetisieren von TGKT durch Kombinieren von TG und KT;
(3) Synthetisieren von TGTGKT durch Binden von TG an den N-Terminus des TGKT;
(4) Synthetisieren von QTGTGKT durch Binden von Q an den N-Terminus von TGTGKT; und
(5) Synthetisieren von X-AQTGTGKT durch Binden eines Alaninderivats (X-A) an den N-Terminus von QTGTGKT.

## Revendications

1. Composé représenté par la Formule Générale suivante :
[Formule Générale] X-AQTGTGKT
(dans la Formule Générale,
A représente l'alanine, Q représente la glutamine, T représente la thréonine, G représente la glycine et K représente la lysine, et le X représente un ou plusieurs éléments sélectionnés dans le groupe consistant en

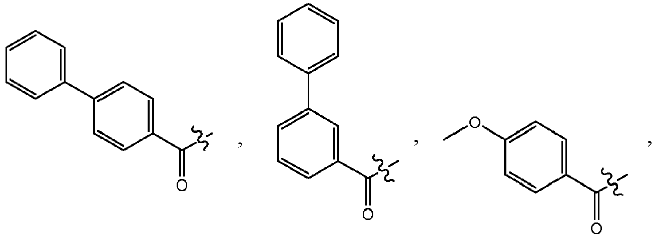

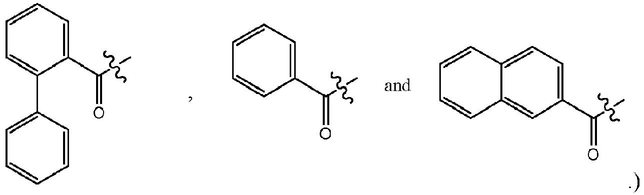

2. Composé destiné à être utilisé dans la prévention ou le traitement du cancer, dans lequel le composé est représenté par la Formule Générale suivante :
[Formule Générale] X-AQTGTGKT
(dans la Formule Générale,
A représente l'alanine, Q représente la glutamine, T représente la thréonine, G représente la glycine et K représente la lysine, et le X représente un ou plusieurs éléments sélectionnés dans le groupe consistant en

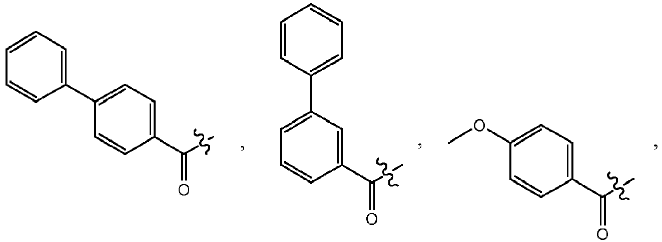

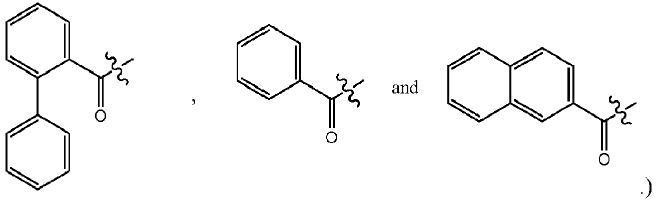

3. Composé destiné à être utilisé selon la revendication 2, dans lequel le cancer est un cancer sélectionné dans le groupe consistant en le cancer du poumon, le cancer du sein, le cancer du sang, le cancer colorectal, le cancer du pancréas et des combinaisons de ceux-ci.

4. Composé destiné à être utilisé selon la revendication 2 ou 3, dans lequel le cancer est un cancer du poumon.

5. Composé destiné à être utilisé selon la revendication 3 ou 4, dans lequel le cancer du poumon est un cancer du poumon non à petites cellules.

6. Composé destiné à être utilisé selon la revendication 2 ou 3, dans lequel le cancer est un cancer du sang.

7. Composé destiné à être utilisé selon la revendication 3 ou 6, dans lequel le cancer du sang est un cancer du sang sélectionné dans le groupe consistant en la leucémie, le lymphome, le myélome multiple et des combinaisons de ceux-ci.

8. Composé destiné à être utilisé selon la revendication 2 ou 3, dans lequel le cancer est un cancer du sein.

9. Composé destiné à être utilisé selon la revendication 2 ou 3, dans lequel le X est un ou plusieurs éléments sélectionnés dans le groupe consistant en 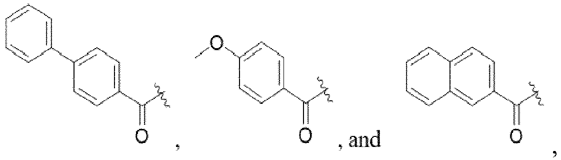 et le cancer est un cancer du pancréas.

10. Composé destiné à être utilisé selon la revendication 2 ou 3, dans lequel le cancer est un cancer colorectal.

11. Composé destiné à être utilisé selon l'une quelconque des revendications 2 à 10, dans lequel, lorsque X est 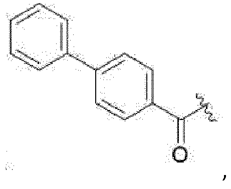 la demi-vie du composé dans le sang humain est de 100 minutes à 150 minutes.

12. Composé destiné à être utilisé selon l'une quelconque des revendications 2 à 10, dans lequel, lorsque X est un ou plusieurs éléments sélectionnés dans le groupe consistant en 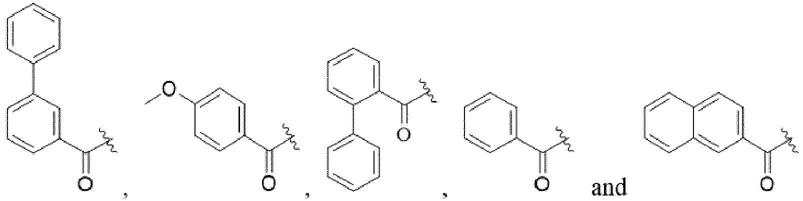 , la demi-vie du composé dans le sang humain est de 45 minutes à 70 minutes.

13. Procédé de préparation d'un oligopeptide X-AQTGTGKT, le procédé comprenant les étapes suivantes :
(A représente l'alanine, Q représente la glutamine, T représente la thréonine, G représente la glycine et K représente la lysine, et
X est l'un sélectionné dans le groupe consistant en 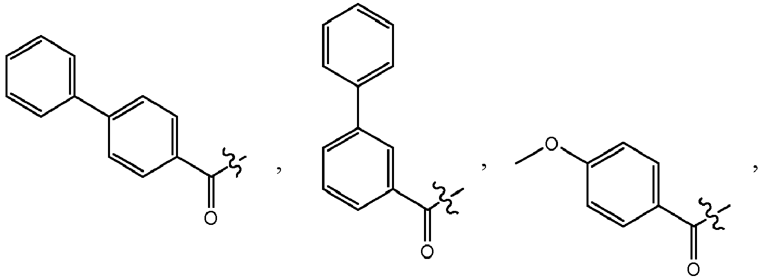 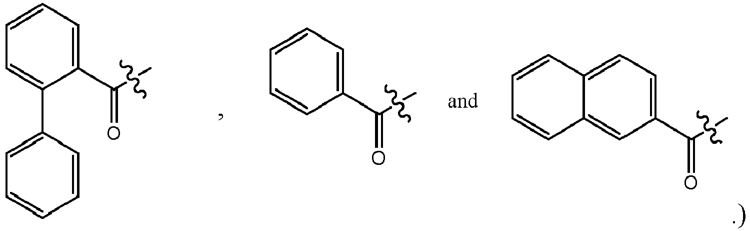
(1) la synthèse de chacun de TG et de KT ;
(2) la synthèse de TGKT en combinant TG et KT ;
(3) la synthèse de TGTGKT en liant TG à l'extrémité N-terminale du TGKT ;
(4) la synthèse de QTGTGKT en liant Q à l'extrémité N-terminale du TGTGKT ; et
(5) la synthèse de X-AQTGTGKT en liant un dérivé d'alanine (X-A) à l'extrémité N-terminale du QTGTGKT.
